# EUROPÄISCHE PATENTSCHRIFT

(11) **EP 2 905 235 B1**
(45) Veröffentlichungstag und Bekanntmachung des Hinweises auf die Patenterteilung: **08.02.2017**
(21) Anmeldenummer: 14195696.1
(22) Anmeldetag: 01.12.2014
(51) Int. Cl.: B65B 55/06, B65B 5/06, A61L 2/04, B32B 3/00, B32B 3/12, A61J 1/16, A61M 5/00, A61L 2/26

(54) **Transport- und Verpackungsbehälter zum Aufnehmen einer Mehrzahl von Behältern für medizinische, pharmazeutische oder kosmetische Anwendungen sowie Verfahren und Verwendungen hiervon**
Transport and packing container for containment of a number of containers for medical, pharmaceutical or cosmetic uses and methods and uses thereof
Récipient d'emballage et de transport destiné à recevoir une pluralité de récipients pour des applications médicales, pharmaceutiques ou cosmétiques et son procédé et ses utilisations

(30) Priorität: 18.12.2013 DE 102013114404
(43) Veröffentlichungstag der Anmeldung: 12.08.2015
(73) Patentinhaber: Schott AG, 55122 Mainz (DE)
(72) Erfinder: Deutschle, Gregor Fritz, 65185 Wiesbaden (DE); Pawlowski, Edgar, 55271 Stadecken-Elsheim (DE); Wassenberg, Jörn, 55130 Mainz (DE); Wissner, Kai, 69493 Hirschberg (DE)
(74) Vertreter: 2K Patentanwälte Blasberg Kewitz & Reichel

(56) Entgegenhaltungen:
- EP-A1- 2 090 324
- EP-A2- 2 659 922
- DE-A1-102012 103 899

## Beschreibung

### Gebiet der Erfindung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen und Karpulen, und betrifft insbesondere die gleichzeitige Halterung einer Mehrzahl von Behältern in einem Transport- und Verpackungsbehälter in solcher Weise, dass diese in einfacher Weise in Abfüll- oder Bearbeitungsanlagen weiter verarbeitet werden können, insbesondere in einem Steril- oder Hitzetunnel, einer Abfüllanlage für flüssige medizinische oder pharmazeutische Anwendungen oder einem Gefriertrockenschrank hierfür.

### Hintergrund der Erfindung

Als Behälter zur Aufbewahrung von und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen, Spritzen oder Karpulen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

GB 2478703 A offenbart eine Haltestruktur zum Halten einer Mehrzahl von Fläschchen für Anwendungen in der Gas- oder Flüssigkeitschromatographie. Die Haltestruktur besteht aus zwei Platten, in denen eine Mehrzahl von Aufnahmen zum Aufnehmen der Fläschchen darin ausgebildet sind und die aufeinander zugeklappt werden können. Die Aufnahmen der beiden Platten sind zueinander versetzt, sodass die Behälter ineinander geschachtelt angeordnet werden, um die Packungsdichte zu verdoppeln, in der aufgefalteten Stellung jedoch einen guten Zugriff auf die Behälter zu ermöglichen. Maßnahmen zur Abdichtung des Innenraums eines von zwei Platten gebildeten Transportbehälters sind nicht offenbart.

Weitere Transport- und Verpackungsbehälter sowie Haltestrukturen werden in US 2007/0272587 A1, US 2011/0132797 A1, FR 2595667 und US 832086 A offenbart.

Ein unmittelbarer Kontakt der Böden der Medikamentenbehälter ist bei den herkömmlichen Haltestrukturen nicht möglich. Dies erschwert jedoch die Weiterverarbeitung der Medikamentenbehälter insbesondere dann, wenn deren Inhalt einer Gefriertrocknung (auch als Lyophilisation oder Sublimationstrocknung bezeichnet) unterzogen werden soll. Ferner ist eine Weiterverarbeitung der Medikamentenbehälter unmittelbar in den Haltestrukturen nicht möglich, da diese dort entweder starr gehalten werden oder für die Weiterverarbeitung nicht in ausreichendem Maß zugänglich sind, weshalb die Medikamentenbehälter für eine Weiterverarbeitung herkömmlich stets aus den Haltestrukturen entnommen werden müssen.

Ein grundlegendes Problem bei der Herstellung und Prozessierung Behältern für medizinische oder pharmazeutische Anwendungen stellen hochtemperaturresistente Verunreinigungen dar, insbesondere Endotoxine, die Lyse der Bakterien sowie eine Reihe weiterer Cytokine induzierende Substanzen (CIS). Endotoxine (Pyrogene) sind Lipopolysaccharide aus den Außenschichten der Zellmembran gram-negativer Bakterien, zum Beispiel E. coli. Endotoxine verursachen bei parenteraler Verabreichung beim Menschen Fieberreaktionen und müssen deshalb unbedingt vermieden werden. Diese Kontaminationen geschehen natürlicherweise zum Beispiel bei bakteriellen Fermentierungsprozessen, können aber auch zum Teil durch den Menschen selbst oder verunreinigte Wassersysteme bei der Herstellung übertragen werden. Endotoxine sind sehr temperaturbeständig und widerstehen Schwankungen des pH-Wertes. Zur Elimination solcher Kontaminationen müssen sehr hohe Temperaturen (insbesondere von über 300°C) eingesetzt werden. In diesem Zusammenhang besteht weiterer Verbesserungsbedarf hinsichtlich Transport- und Verpackungsbehältern oder Trägern hiervon, welche die Behälter während einer Prozesssierung derselben bei hohen Temperaturen aufnehmen bzw. halten und hinsichtlich Verfahren zur Entfernung solcher Kontaminationen in Behältern durch Prozessierung bei hohen Temperaturen in einfacher und kostengünstiger Weise.

Als vorteilhaft zur Verhinderung derartiger Kontaminationen in Behältern für Substanzen für medizinische oder pharmazeutische Anwendungen, insbesondere solchen aus Glas, hat sich das Einbrennen von Silikonen zur Fixierung des Silikons an der Glasoberfläche erwiesen. Hierzu sind hohe Temperaturen von über 300 °C notwendig. Dazu ist es vorteilhaft die Prozessierung des Glaskörpers unter Reinraumbedingungen durchzuführen. Die Silikonisierung schafft eine Schutzschicht für Medikamente auf Basis von empfindlichen Molekülen, die auf den Glasbehälter reagieren können. In diesem Zusammenhang besteht weiterer Verbesserungsbedarf hinsichtlich Transport- und Verpackungsbehältern oder Trägern hiervon, welche die Behälter während einer Prozessierung derselben und während des Einbrennens von Silikonschichten bei hohen Temperaturen aufnehmen bzw. halten und hinsichtlich Verfahren zur Prozessierung derselben und während des Einbrennens von Silikonschichten bei hohen Temperaturen in einfacher und kostengünstiger Weise.

### Zusammenfassung der Erfindung

Aufgabe der vorliegenden Erfindung ist es, einen verbesserten Transport- und Verpackungsbehälter bereitzustellen, der eine rasche Entnahme oder Prozessierung der in dem Transport- und Verpackungsbehälter aufgenommenen Behälter in sofort füllfähiger Form in sog. Ready-to-Fill-Prozessen ermöglicht. Insbesondere sollen die Behälter einfach und kostengünstig steril verpackt, wieder entpackt und weiterverarbeitet werden können.

Weitere Aufgaben der vorliegenden Erfindung sind die Bereitstellung eines Verfahrens sowie von Verwendungen, um die Behälter in sofort füllfähiger Form in sog. Ready-to-Fill-Prozessen zu prozessieren, wobei die Behälter einfach und kostengünstig steril verpackt, wieder entpackt und weiterverarbeitet werden sollen.

Diese Aufgaben werden gemäß der vorliegenden Erfindung durch einen Transport- und Verpackungsbehälter mit den Merkmalen nach Anspruch 1 und durch ein Verfahren nach Anspruch 13 gelöst. Weitere vorteilhafte Ausführungsformen sind Gegenstand der rückbezogenen Unteransprüche.

Gemäß der vorliegenden Erfindung wird ein Transport- und Verpackungsbehälter zum Aufnehmen einer Mehrzahl von zylindrischen Behältern für Substanzen für medizinische, pharmazeutische oder kosmetische Anwendungen bereitgestellt, der zumindest zwei Segmente aufweist, die jeweils separat gehandhabt werden können und die zusammengesetzt oder zusammengesteckt werden können, um gemeinsam den Transport- und Verpackungsbehälter mit einem Innenraum auszubilden, in welchem die Behälter abgedichtet gegen die Umgebung aufbewahrt werden können. In dem Transport- und Verpackungsbehälter weist ein erstes der zumindest zwei Segmente einen Boden zum unmittelbaren oder mittelbaren Abstützen der Mehrzahl von Behältern auf dem Boden auf. Ferner sind in dem Transport- und Verpackungsbehälter Positionierungsmittel vorgesehen, um die Mehrzahl von Behältern im Innenraum des Transport- und Verpackungsbehälters in einer regelmäßigen Anordnung so zu positionieren, dass eine Berührung von unmittelbar benachbarten Behältern verhindert ist. Dabei weist zumindest eines der Segmente elastische Dichtungsmittel auf, sodass die Segmente zu dem Transport- und Verpackungsbehälter zusammengesetzt oder zusammengesteckt werden können, sodass der Innenraum des Transport- und Verpackungsbehälters gegen die Umgebung abgedichtet ist.

Somit sind die Behälter an vorbestimmten Position im Innenraum des Transport- und Verpackungsbehälters zuverlässig und kollisionsfrei angeordnet, sodass die Behälter an automatisierte Prozessierungsstationen in einfacher Weise übergeben und/oder in diesen weiter verarbeitet werden können. Diese Weiterverarbeitung kann außerhalb des Transport- und Verpackungsbehälters erfolgen, insbesondere während die Mehrzahl von Behältern in einer Haltestruktur, die in den Transport- und Verpackungsbehälter eingesetzt ist, gehalten sind, oder kann erfolgen, während die Behälter in einem Segment des Transport- und Verpackungsbehälters angeordnet sind, beispielsweise in einer Haltestruktur, die auf dem Boden des Segments angeordnet ist. Somit ist es erfindungsgemäß möglich, nahezu beliebig geformte Behälter, insbesondere Fläschchen (Vials), Spritzen (syringe) und Doppelkammer-Spritzen (dual-chamber syringe) oder Karpulen (cartridges) und Doppelkammer-Karpulen (dual chamber cartridges), sowie Vartridges, mit unterschiedlichen Größen (Länge und Durchmesser) sicher und ohne Glaskontakt zu transportieren.

Die Dichtungsmittel ermöglichen dabei eine zuverlässige Abdichtung des Innenraums des Transport- und Verpackungsbehälters gegen die Umgebung, die grundsätzlich auch für eine sterile Aufbewahrung der Behälter im Innenraum ausgelegt sein kann. Bevorzugt können die Segmente mehrfach auseinander gezogen oder genommen werden und wieder zu dem Transport- und Verpackungsbehälter zusammen geschoben oder zusammen gesteckt werden, beispielsweise zur vorübergehenden Entnahme von Behältern, zu deren Weiterverarbeitung in einer Prozessstation und zu deren anschließender Verpackung, beispielsweise für einen sterilen Weitertransport zu einer weiteren Prozessstation oder zum Endkunden. Während nach dem Stand der Technik hierzu das Aufbrechen von Versiegelungen, wie beispielsweise sterilen Schutzfolien, notwendig war, kann der erfindungsgemäße Transport- und Verpackungsbehälter nahezu beliebig oft geöffnet und wieder verschlossen werden.

Die Segmente können nahezu beliebig geformt sein, solange diese im zusammengesetzten Zustand einen geschlossen ausgebildeten Transport- und Verpackungsbehälter ausbilden können. Als besonders geeignet haben sich Segmentformen mit plattenförmigen Seitenwänden und/oder Böden erwiesen, wobei die Seitenwände im zusammengesetzten Zustand der Segmente bevorzugt unmittelbar die Seitenwände des Transport- und Verpackungsbehälters ausbilden.

Gemäß einer bevorzugten weiteren Ausführungsform sind die Segmente jeweils als ausziehbare Schubfächer jeweils bestehend aus drei Seitenwänden und einem Boden ausgebildet, sodass sich der Transport- und Verpackungsbehälter durch seitlichen Schub der Schubfächer öffnen und wieder verschließen lässt.

Grundsätzlich bevorzugt werden dabei Ausführungsformen, bei denen insgesamt zwei Segmente in der Art von Schubfächern einen insgesamt kastenförmigen Transport- und Verpackungsbehälter ausbilden. Grundsätzlich möglich ist jedoch auch, dass bei einer Mehrzahl von Segmenten, die jeweils in der Art eines Schubfachs ausgebildet sind, jeweils ein Segment über einem anderen Segment angeordnet wird und diese ineinander geschoben werden, um gemeinsam einen Transport- und Verpackungsbehälter in einer gestapelten Anordnung von mehreren Segmenten auszubilden, aus der einzelne schubfachartige Segmente wieder abgezogen werden können.

Zu diesem Zweck sind erfingdungsgemäß an den Segmenten korrespondierend zueinander ausgebildete Führungsschienen und Führungsausnehmungen vorgesehen, welche die Segmente beim Ineinanderschieben zum Zusammensetzen des Transport- und Verpackungsbehälters geeignet führen. Der Transport- und Verpackungsbehälter kann somit in besonders einfacher Weise auseinander genommen und wieder zusammengesetzt werden, nämlich durch seitliches Einschieben der Segmente, was sowohl manuelle als auch automatisiert erfolgen kann, beispielsweise durch Roboter in einer Prozessstation.

Dabei dienen die Führungsschienen und Führungsausnehmungen der Segmente erfingdungsgemäß auch der gewünschten Abdichtung des Transport- und Verpackungsbehälters. Hierzu sind die Führungsschienen oder Führungsausnehmungen mit geeigneten Dichtungslippen umgeben sein, die ein Eindringen von Verunreinigungen in den Innenraum des Transport- und Verpackungsbehälters verhindern. Oder die Führungsschienen und Führungsausnehmungen können mit einer elastischen Kunststoffauflage versehen sein, beispielsweise ausgebildet in einer 2K-Spritzgusstechnik, sodass die elastische Kunststoffauflage elastisch gegen gegenüberliegende Seitenwände der Führungsschienen oder Führungsausnehmungen des anderen Segment drückt, um den Innenraum des Transport- und Verpackungsbehälters gezielt auch in diesen Bereichen abzudichten.

Zur zusätzlichen Abdichtung des Innenraums des Transport- und Verpackungsbehälters kann gemäß einer weiteren Ausführungsform am unteren Rand der Seitenwände des jeweiligen Segments zumindest eine weitere Ausnehmung oder ein weiterer Vorsprung ausgebildet sein, die oder der als zusätzliches Dichtungsmittel wirkt und sich in Längsrichtung und parallel zu den an den Seitenwänden vorgesehenen und korrespondierend zueinander ausgebildeten Führungsschienen und Führungsausnehmungen erstreckt. Damit ist insbesondere ein Eindringen von Verunreinigungen in den Innenraum des Transport- und Verpackungsbehälters am unteren Rand des Transport- und Verpackungsbehälters weiter verhindert. Dabei können auch die weitere Ausnehmung bzw. der weiterer Vorsprung in der vorstehend beschriebenen Weise mit einer elastischen Kunststoffauflage versehen sein, beispielsweise ausgebildet in einer 2K-Spritzgusstechnik, sodass die weitere Ausnehmung bzw. der weiterer Vorsprung gegen die jeweils zugeordnete Führungsschiene oder Führungsausnehmung elastisch verspannt ist, um den Innenraum des Transport- und Verpackungsbehälters gezielt auch in diesen Bereichen abzudichten.

Um den Transport- und Verpackungsbehälter rasch öffnen und zuverlässig wieder verschließen zu können, sind die Segmente gemäß einer weiteren Ausführungsform unmittelbar miteinander verrastbar, um den Transport- und Verpackungsbehälter auszubilden. Dies kann grundsätzlich auch durch auf der Außenseite des Transport- und Verpackungsbehälters vorgesehene Verriegelungsmittel bewirkt werden. Gemäß einer bevorzugten weiteren Ausführungsform sind jedoch solche Verriegelungsmittel unmittelbar an den Segmenten vorgesehen oder werden diese durch geeignete Formgestaltung unmittelbar von diesen ausgebildet, insbesondere von den miteinander zusammenwirkenden Rändern bzw. Endbereichen der Seitenwände der Segmente, um die Segmente im zusammengesetzten oder zusammengesteckten Zustand miteinander zu verriegeln. Beim Zusammensetzten oder Zusammenstecken der zumindest zwei Segmente geraten so die Verriegelungsmittel schließlich in einen mechanischen Eingriff, um die vorgenannte Verriegelung zu bewirken. Zweckmäßig sind hierzu an den Rändern bzw. Endbereichen der Seitenwände der Segmente formschlüssig miteinander zusammen wirkende Vorsprünge und korrespondierend dazu ausgebildete Ausnehmungen ausgebildet, die miteinander verrastet werden können und bevorzugt elastisch gegeneinander vorgespannt sind, insbesondere bewirkt durch die Materialeigenschaften der Seitenwände in diesen Bereich selbst oder mittels elastischer Rückstellelemente, insbesondere Federn oder dergleichen.

Gemäß der Erfindung sind an den Segmenten selbst elastische Dichtungsmittel vorgesehen, um den Innenraum des Transport- und Verpackungsbehälters gegen die Umgebung hin abzudichten. Dies kann durch geeignete Formgestaltung der beim Zusammensetzen oder Zusammenstecken der Segmente miteinander in Anlage gelangenden Ränder oder Endbereiche der Seitenwände der Segmente erfolgen, insbesondere indem die einander gegenüber liegenden Ränder oder Endbereiche der Seitenwände exakt korrespondierend zueinander ausgebildet sind, sodass in diesen Bereichen keine unerwünschten Luftspalte verbleiben können. Vorteilhaft ist, wenn in diesen Bereichen die Ränder oder Endbereiche der Seitenwände über eine gewisse Elastizität verfügen, um diese Bereiche im zusammengesetzten oder zusammengesteckten Zustand der Segmente in gewissem Maße elastisch gegeneinander zu verspannen oder diese leicht verformt gegeneinander gedrückt zu halten. Dies kann durch gezielte Wahl der Materialeigenschaften der Seitenwände in diesen Bereichen erzielt werden, oder durch gezieltes Anordnen von elastischen Dichtungslippen in diesen Bereichen. Solche elastischen Dichtungslippen können grundsätzlich in Gestalt von separaten Dichtungselementen in Nuten, die in den vorgenannten Bereichen angeordnet sind, eingebracht sein, können jedoch auch in einer 2K-Spritzgusstechnik aus einem weicheren elastischen Kunststoff in diesen Bereichen an die Segmente angespritzt sein.

Gemäß einer weiteren Ausführungsform sind die Positionierungsmittel unmittelbar an zumindest einem der Segmente ausgebildet, insbesondere einstückig mit diesem ausgebildet, was sich insbesondere bei Herstellung der Segmente durch Spritzgussverfahren aus Kunststoff einfach realisieren lässt. Hierzu können geeignet einstückig mit dem jeweiligen Segment ausgebildete Zapfen oder Wandabschnitte unter einem rechten Winkel von der Innenseite bzw. dem Boden des jeweiligen Segments so abragen, um eine Berührung von unmittelbar benachbarten Behältern zu verhindern, wenn diese in dem Transport- und Verpackungsbehälter aufgenommen sind.

Gemäß einer weiteren Ausführungsform ist das Positionierungsmittel durch einen Träger bzw. eine Haltestruktur ausgebildet, der als separates Bauteil in den Transport- und Verpackungsbehälter eingesetzt und aus diesem wieder herausgenommen werden kann. In dem Träger bzw. der Haltestruktur ist eine Mehrzahl von Aufnahmen ausgebildet, die so ausgelegt sind, dass die Behälter in die zugeordneten Aufnahmen von oben oder von unten her eingeführt werden können.

Bevorzug ist die Haltestruktur bzw. der Träger so ausgelegt, dass die Behälter (container) darin reibschlüssig bzw. geklemmt gehalten. Zur kraft- bzw. reibschlüssigen Halterung von zylindrischen Behältern dienen Aufnahmen, die bevorzugt umlaufend ausgebildet sind und die sich in Längsrichtung der Behälter erstrecken. Kraft- bzw. reibschlüssige Verbindungen setzen bekanntermaßen nur eine ausreichende Normal-Kraft auf die miteinander zu verbindenden Flächen voraus. Die gegenseitige Verschiebung zwischen Behälter und Haltestruktur ist also verhindert solange die durch die Haftreibung zwischen Haltestruktur und Behälter bewirkte Gegenkraft nicht überschritten wird. Der Kraft- bzw. Reibschluss ist verloren und die Flächen rutschen aufeinander, wenn die tangential wirkende Last-Kraft größer als die Haftreibungs-Kraft ist. Letzteres ist jedoch bei den vergleichsweise geringen Gewichten der in der Haltestruktur aufzunehmenden Behälter unwahrscheinlich, kann jedoch ausgenutzt werden, um die Behälter, während diese in der Haltestruktur aufbewahrt sind, von einer ersten Stellung axial in eine zweite Stellung zu verschieben, in welcher diese weiterverarbeitet werden können, beispielsweise deren Öffnungen mit einem Stopfen verschlossen werden oder auf den Stopfen ein äußerer Verschluss (beispielsweise Bördelkappe oder Krampe), häufig aus Aluminiumblech, aufgebracht wird.

Der Kraft- bzw. Reibschluss erfolgt zweckmäßig entweder unterhalb des aufgeweiteten oberen Randes der Behälter, d.h. in ihrem verengten Hals- bzw. Nackenbereich unterhalb des oberen Rands, oder im Bereich der zylindrischen Seitenwand. Einer bodenseitigen Abstützung der Behälter bedarf es hierzu grundsätzlich nicht, sodass ein Zugriff auf die Unterseiten (Böden) der in der Haltestruktur gehaltenen Behälter grundsätzlich möglich ist. Dies ermöglicht, dass die Behälter, während diese in der Haltestruktur aufgenommen sind, weiterverarbeitet werden können. Mit anderen Worten, die Behälter können in den Haltestrukturen chargenweise weiter verarbeitet werden, wenn diese aus dem Transport- und Verpackungsbehälter herausgenommen sind, bleiben jedoch während der Weiterverarbeitung weiterhin zuverlässig und kollisionsfrei in den Haltestrukturen gehalten, was erhebliche Geschwindigkeitsvorteile und Vorteile bei der Automatisierung von Weiterverarbeitungsanlagen ermöglicht und so insgesamt zu noch wirtschaftlicheren und kostengünstigeren Prozessen führt. Die Behälter können insbesondere in der Haltestruktur axial angehoben oder gedreht werden. Die Behälter können gemäß weiteren Ausführungsformen auch in der Haltestruktur lyophilisiert werden, da ein direkter Bodenkontakt zu einem Kühlfinger des Gefriertrockenschranks möglich ist. Böden oder Köpfe der Behälter sind in einfacher Weise auf einer Höhe fixierbar, sodass sämtliche Böden in einer gemeinsam aufgespannten Ebene angeordnet werden können, was den unmittelbaren Kontakt zu flächigen Weiterverarbeitungstationen, insbesondere zu einem Kühlfinger bzw. einer Kühlhorde eines Gefriertrockenschranks ermöglicht.

Die Haltestruktur ermöglicht dabei zweckmäßig die Entnahme der Behälter nach oben oder unten. Da die Position des Kraft- bzw. Reibschlusses zwischen Behälter und Haltestruktur einfach variiert werden kann, ist die Haltestruktur sehr flexibel auch für Behälter mit unterschiedlichen Außenabmessungen einsetzbar, solange eine ausreichende Normalkraft für den Reibschluss gewährleistet werden kann. Insbesondere können die Behälter in der Haltestruktur in axialer Richtung einfach verschoben werden, sodass Behälter mit unterschiedlichen Höhen in derselben Haltestruktur gehalten werden können. Die axiale Verschiebbarkeit der Behälter in der Haltestruktur ermöglicht auch einen einfachen Toleranzausgleich.

Gemäß einer weiteren Ausführungsform kann die Öffnungsweite bzw. der Reibschluss von sämtlichen Aufnahmen der Haltestruktur durch koordiniertes Verstellen der Seitenwände der Aufnahmen gemeinsam bzw. koordiniert verstellt werden. Zu diesem Zweck sind sämtliche Aufnahmen der Haltestruktur bevorzugt miteinander mechanisch gekoppelt, sodass eine Verstellung oder Verformung der Haltestruktur zu einer koordinierten Verstellung der Öffnungsweite sämtlicher Aufnahmen führt. Dabei ist die koordinierte Verstellung so ausgelegt, dass die Seitenwände der Aufnahmen zwischen einer ersten Stellung, in welcher die Behälter mit geringem Kraftaufwand in die Öffnungen oder Aufnahmen der Haltestruktur einführbar sind, und einer zweiten Stellung koordiniert verstellbar sind, in welcher die Behälter in den Öffnungen oder Aufnahmen der Haltestruktur reibschlüssig fixiert sind.

Gemäß einer weiteren Ausführungsform ist zumindest ein Boden des Transport- und Verpackungsbehälters aus einem hochtemperaturfesten Kunststoff ausgebildet, insbesondere aus einem thermoplastischen Kunststoff, der Temperaturen von bis zu 330°C und bevorzugter bis 350 °C resistiert, insbesondere aus Polyimide (PI), Polyetherketon (PEK), Polyetheretherketon (PEEK), Polyetherketonketon (PEKK) oder Polyetherketonetherketonketon (PEKEEK). Der Boden kann hierzu auch von den restlichen Segmenten des Transport- und Verpackungsbehälters vorübergehend abgenommen werden. Dies ermöglicht eine Verarbeitung der Behälter bei sehr hohen Temperaturen.

Gemäß einer alternativen Ausführungsform ist zumindest ein Boden des Transport- und Verpackungsbehälters aus einem Metall ausgebildet ist, wobei das Metall zweckmäßig mit einem Kunststoff beschichtet ist, insbesondere aus einem thermoplastischen Kunststoff, der Temperaturen von bis zu 330°C und bevorzugter bis 350 °C resistiert, insbesondere aus Polyimide (PI), Polyetherketon (PEK), Polyetheretherketon (PEEK), Polyetherketonketon (PEKK) oder Polyetherketonetherketonketon (PEKEEK). Der Boden kann hierzu auch von den restlichen Segmenten des Transport- und Verpackungsbehälters vorübergehend abgenommen werden.

Gemäß einer weiteren alternativen Ausführungsform ist zumindest ein Boden des Transport- und Verpackungsbehälters aus einem Metall ausgebildet ist, wobei das Metall mit einem Basisbeschichtungsmaterial bestehend aus SiO₂, TiO₂, Al₂O₃ oder ZrO₂ auf die Oberfläche aufgebracht und anschließend getempert oder gebrannt wird. Geeignete Beschichtungsverfahren sind beispielsweise Sputtern, PVD, CVD, Sol-Gel-Beschichtung oder Sprühbeschichtung.

Dies ermöglicht eine Verarbeitung der Behälter bei sehr hohen Temperaturen, insbesondere die thermische Zerstörung von Endotoxinen, die Lyse der Bakterien sowie eine Reihe weiterer Cytokine induzierende Substanzen (CIS), sowie das Einbrennen von Silikonen in die Behälter, insbesondere in aus Glas ausgebildete Behälter, aber auch eine Gefriertrocknung von in den Behältern aufgenommenen Substanzen, während die Behälter in dem Transport- und Verpackungsbehälter, einem Segment davon oder einem Träger, die darin aufgenommen oder eingesetzt ist, aufgenommen sind. Vorteilhaft ist, dass die Behälter zur Prozessierung nicht extra aus dem Transport- und Verpackungsbehälter oder einem darin aufgenommen oder eingesetzten Träger entnommen und vereinzelt werden müssen, sondern unmittelbar prozessiert werden können. Ein erneutes Einstecken oder Einsetzen der Behälter in die Positionierungsmittel des Transport- und Verpackungsbehälters oder eines darin aufgenommen oder eingesetzten Trägers ist somit erfindungsgemäß nicht erforderlich.

Gemäß einer weiteren Ausführungsform sind Seitenwände des Transport- und Verpackungsbehälters zumindest abschnittsweise mit Durchbrechungen versehen, die mittels einer aufgeklebten oder in anderer Weise aufgebrachten und bevorzugt gasdurchlässigen Kunststofffolie verschlossen sind, insbesondere einem Geflecht aus Kunststofffasern, beispielsweise aus Polypropylen-Fasern (PP) oder einer Tyvek®-Schutzfolie. Die Durchbrechungen können insbesondere als vergleichsweise große Öffnungen beispielsweise auf Seitenwänden des Transport- und Verpackungsbehälters angeordnet sein. Oder die Seitenwände sind abschnittsweise mit einer durchlässigen, gitterförmigen Struktur versehen, auf die die Kunststofffolie dann zur Abdeckung geeignet aufgebracht wird, um diese zu verschließen. Mittels der Kunststofffolie kann somit der Transport- und Verpackungsbehälter steril verschlossen werden. Durch die Durchbrechungen kann somit ein Gas zum Sterilisieren des Innenraums des Transport- und Verpackungsbehälters einströmen. Mittels der Folie ist dabei sichergestellt, dass der Innenraum des Transport- und Verpackungsbehälters gegen die Umgebung steril abgedichtet ist.

Gemäß einem weiteren Gesichtspunkt der vorliegenden Erfindung wird somit ein Verfahren zur thermischen Behandlung von Behältern für Substanzen für medizinische, pharmazeutische oder kosmetische Anwendungen bereitgestellt, insbesondere unter Verwendung eines Transport- und Verpackungsbehälters, wie vorstehend beschrieben, und/oder eines Trägers mit Positionierungsmitteln, wie vorstehend beschrieben, wobei der Boden des Transport- und Verpackungsbehälters mit einer Mehrzahl von darin aufgenommenen zylindrischen Behältern oder der Träger mit einer Mehrzahl von diesem gehaltenen zylindrischen Behältern in einer Wärme-Prozessierungsstation (einer die Behälter bei hohen Temperaturen behandelnden Station), insbesondere in einem Heißofen oder einem Heißtunnel, mit Temperaturen von bis zu 330°C und bevorzugter bis zu 350 °C prozessiert wird. Vorteilhaft ist, dass die Behälter zur Prozessierung nicht extra aus dem Transport- und Verpackungsbehälter oder einem darin aufgenommen oder eingesetzten Träger entnommen und vereinzelt werden müssen, sondern unmittelbar prozessiert werden können. Ein erneutes Einstecken oder Einsetzen der Behälter in die Positionierungsmittel des Transport- und Verpackungsbehälters oder eines darin aufgenommen oder eingesetzten Trägers ist somit erfindungsgemäß nicht erforderlich.

Gemäß einer weiteren Ausführungsform weist das Verfahren die weiteren Schritten auf: Anordnen der Mehrzahl von Behältern in dem Transport- und Verpackungsbehälter, sodass die Behälter von den Positionierungsmitteln im Innenraum des Transport- und Verpackungsbehälters in einer regelmäßigen Anordnung so positioniert sind, dass eine Berührung von unmittelbar benachbarten Behältern verhindert ist; und Prozessierung der Mehrzahl von Behältern, während diese in dem Transport- und Verpackungsbehälter oder in einem Segment davon angeordnet sind, in einer Wärme-Prozessierungsstation, insbesondere in einem Heißofen oder einem Heißtunnel, mit Temperaturen von bis zu 330°C und bevorzugter bis zu 350°C. Zunächst werden die Behälter, die zur Aufbewahrung der Substanzen für die medizinische oder pharmazeutische Anwendungen bestimmt sind, in einen Transport- und Verpackungsbehälter eingesetzt, wie vorstehend beschrieben. In diesem Zustand können die Behälter steril verpackt transportiert oder aufbewahrt werden, da der Transport- und Verpackungsbehälter hierfür ausgelegt ist. Die Prozessierung bei den sehr hohen Temperaturen der Behälter kann grundsätzlich ohne weiteres Öffnen des Transport- und Verpackungsbehälters erfolgen. Gemäß bevorzugten Ausführungsformen wird jedoch der Transport- und Verpackungsbehälter so auseinander genommen, dass die in zumindest einem seiner Segmente angeordneten Behälter, ggf. in einem in dem zumindest einem Segment angeordneten Träger, für eine Weiterverarbeitung zugänglich sind, beispielsweise zur Befüllung der Behälter. Nach der Prozessierung bei den sehr hohen Temperaturen wird der Transport- und Verpackungsbehälter durch Zusammensetzen oder Zusammenstecken seiner Segmente wieder geschlossen, sodass erneut ein Zustand ermöglicht ist, in dem die Behälter in dem Transport- und Verpackungsbehälter steril verpackt transportiert oder aufbewahrt werden.

Gemäß einer weiteren Ausführungsform werden hierzu die Segmente so zusammengesetzt oder zusammengesteckt, um den Transport- und Verpackungsbehälter erneut auszubilden, sodass die Dichtungsmittel den Innenraum des Transport- und Verpackungsbehälters gegen die Umgebung abdichten.

Gemäß einer weiteren Ausführungsform weist das Verfahren die folgenden weiteren Schritte auf: Öffnen des Transport- und Verpackungsbehälters durch Verschieben oder Freigeben von zumindest einem Segment der Mehrzahl von Segmenten des Transport- und Verpackungsbehälters, sodass der Innenraum zur Entnahme des Trägers mit der Mehrzahl von diesem gehaltenen Behältern zugänglich ist; Herausnehmen des Trägers mit der Mehrzahl von diesem gehaltenen Behältern aus dem Transport- und Verpackungsbehälter oder zumindest einem Segment hiervon; Prozessierung der Mehrzahl von Behältern, während diese von dem Träger gehalten sind, in einer Wärme-Prozessierungsstation, insbesondere in einem Heißofen oder einem Heißtunnel, mit Temperaturen von bis zu 330°C und bevorzugter bis zu 350°C; erneutes Anordnen des Trägers mit der Mehrzahl von diesem gehaltenen Behältern in oder auf zumindest einem der Segmente; und Zusammensetzen oder Zusammenstecken der Segmente, um den Transport- und Verpackungsbehälter, sodass die Dichtungsmittel den Innenraum des Transport- und Verpackungsbehälters gegen die Umgebung abdichten. Die Behälter können somit unmittelbar und während diese in den Aufnahmen gehalten sind, prozessiert werden. Ein erneutes Einstecken oder Einsetzen der Behälter in die Positionierungsmittel des Trägers ist somit erfindungsgemäß nicht erforderlich.

Gemäß einer weiteren Ausführungsform erfolgt bei dem Verfahren die Prozessierung der Mehrzahl von Behältern in dem Heißofen oder Heißtunnel mit Temperaturen von bis zu 330°C und bevorzugter bis zu 350°C derart, dass Endotoxine und/oder die Lyse von Bakterien und/oder Cytokine induzierende Substanzen (CIS) in den Behältern thermisch entfernt werden können. Ein erneutes Einstecken oder Einsetzen der Behälter in die Positionierungsmittel des Transport- und Verpackungsbehälters oder eines darin aufgenommen oder eingesetzten Trägers ist somit erfindungsgemäß nicht erforderlich.

Gemäß einer weiteren Ausführungsform erfolgt die Prozessierung der Mehrzahl von Behältern in dem Heißofen oder Heißtunnel mit Temperaturen von bis zu 330°C und bevorzugter bis zu 350°C derart, dass Silikonschichten auf Innenoberflächen der Behälter, insbesondere auf einem Boden und unterem Bereich derselben, eingebrannt werden können. Ein erneutes Einstecken oder Einsetzen der Behälter in die Positionierungsmittel des Transport- und Verpackungsbehälters oder eines darin aufgenommen oder eingesetzten Trägers ist somit erfindungsgemäß nicht erforderlich.

Ein weiterer Gesichtspunkt der vorliegenden Erfindung betrifft die Verwendung eines Transport- und Verpackungsbehälters mit einer Mehrzahl von darin in einem Träger davon aufgenommenen Behältern, wie vorstehend beschrieben, zur Prozessierung mit Temperaturen von bis zu 330°C und bevorzugter bis zu 350°C in einem Heißofen oder einem Heißtunnel.

Gemäß einer weiteren Ausführungsform erfolgt die Prozessierung mit Temperaturen von bis zu 330°C und bevorzugter bis zu 350°C in dem Heißofen oder Heißtunnel derart, dass Endotoxine und/oder die Lyse von Bakterien und/oder Cytokine induzierende Substanzen (CIS) in den Behältern thermisch entfernt werden können oder Silikonschichten auf Innenoberflächen der Behälter, insbesondere auf einem Boden und unterem Bereich derselben, eingebrannt werden können. Ein erneutes Einstecken oder Einsetzen der Behälter in die Positionierungsmittel des Transport- und Verpackungsbehälters oder eines darin aufgenommen oder eingesetzten Trägers ist somit erfindungsgemäß nicht erforderlich.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a - 1c: einen Transport- und Verpackungsbehälter gemäß einer ersten Ausführungsform der vorliegenden Erfindung;
- Fig. 2a - 2b: einen Transport- und Verpackungsbehälter gemäß einer weiteren Ausführungsform der vorliegenden Erfindung;
- Fig. 3: einen Transport- und Verpackungsbehälter gemäß einer weiteren Ausführungsform der vorliegenden Erfindung;
- Fig. 4a - 4d: einen Transport- und Verpackungsbehälter gemäß einer weiteren Ausführungsform der vorliegenden Erfindung, wobei auch Einzelheiten der Dichtungsmittel dargestellt sind;
- Fig. 5a - 5c: einen Transport- und Verpackungsbehälter gemäß einer weiteren Ausführungsform der vorliegenden Erfindung;
- Fig. 6a - 8d: weitere Haltestrukturen, die zum Einsatz in einen Transport- und Verpackungsbehälter gemäß der vorliegenden Erfindung geeignet sind;
- Fig. 9 - 10: Beispiele für Verfahren, bei denen Behälter für Substanzen für medizinische oder pharmazeutische Anwendungen bei sehr hohen Temperaturen gemäß der vorliegenden Erfindung prozessiert werden, während diese in einem Träger aufgenommen sind; und
- Fig. 11: ein schematisches Flussdiagramm für ein Verfahren gemäß der vorliegenden Erfindung, wie in den Figuren 9 und 10 gezeigt.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Ein Transport- und Verpackungsbehälter mit oder ohne eine zusätzliche Haltestruktur dient gemäß der vorliegenden Erfindung, wie nachfolgend beschrieben, der gleichzeitigen Halterung einer Mehrzahl von Behältern zur Aufbewahrung von Substanzen für medizinische, pharmazeutische oder kosmetische Anwendungen in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen.

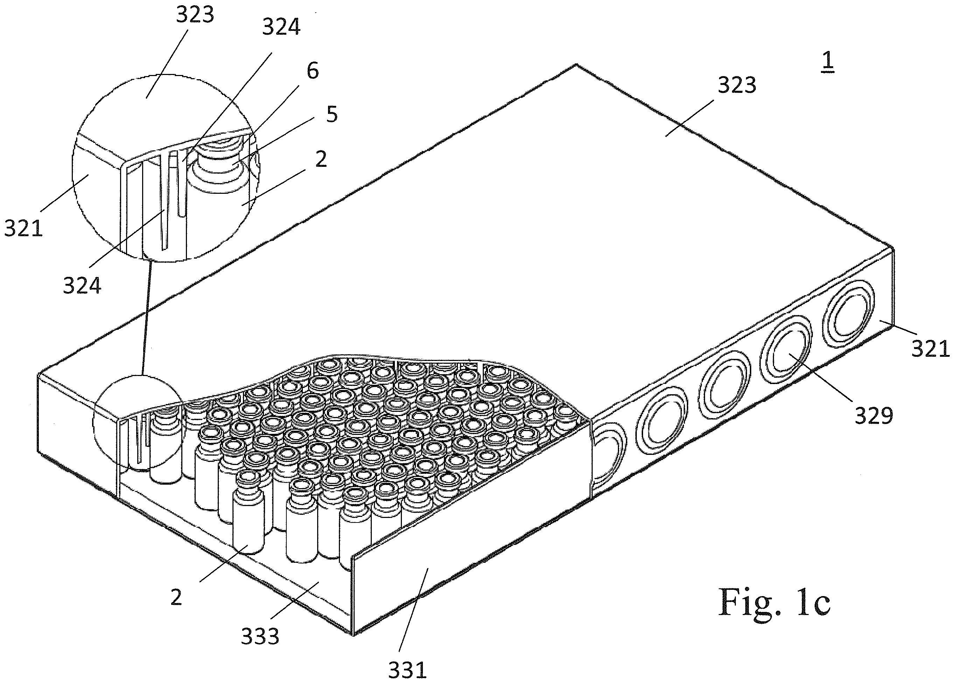

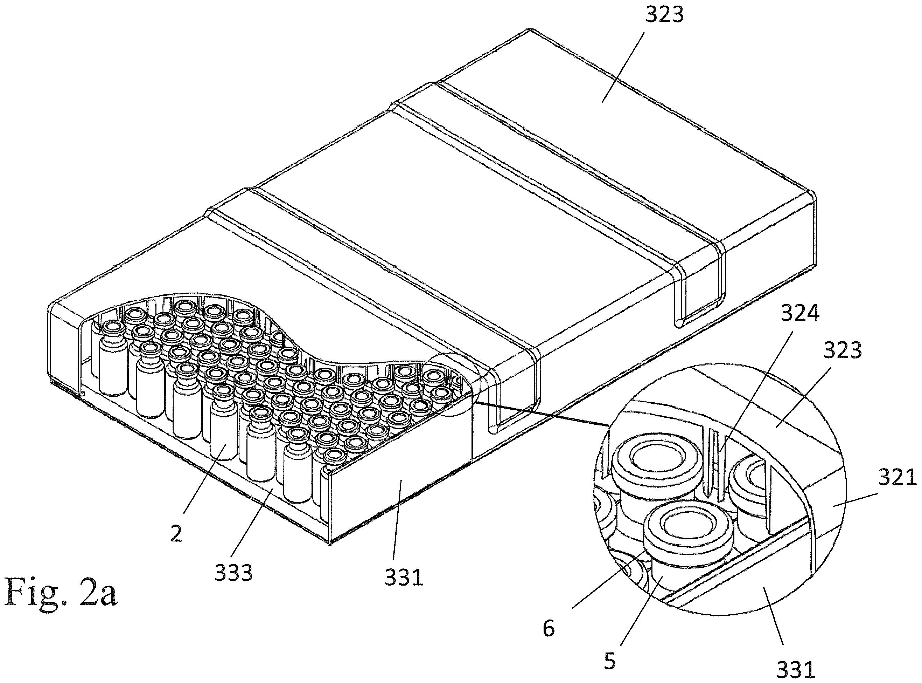

Ein Beispiel für derartige Behälter in Gestalt von Fläschchen (englisch: vial) ist in den stark vergrößerten Einsätzen in den Figuren 1c und 2a schematisch dargestellt. Die Fläschchen 2 weisen eine zylindrische Grundform auf, mit einer zylinderförmigen Seitenwand 4 mit - im Rahmen der Toleranzen - konstantem Innen- und Außendurchmesser, die von einem flach ausgebildeten Flaschenboden senkrecht abragt und nahe dem oberen offenen Ende des Fläschchens in einen verengten Halsabschnitt 5 von vergleichsweise geringer axialer Länge und anschließend in einen verbreiterten oberen Rand 6 übergeht, der einen größeren Außendurchmesser aufweist als der zugeordnete Halsabschnitt 5 und zur Verbindung mit einem Verschlusselement ausgelegt ist. Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Medikamentenbehälter im Sinne der vorliegenden Anmeldung sind Ampullen, Karpulen oder Spritzen- oder Injektionsbehältnisse.

Im Sinne der vorliegenden Erfindung dienen derartige Behälter (container) zur Aufbewahrung von Substanzen oder Wirkstoffen für medizinische, pharmazeutische oder kosmetische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

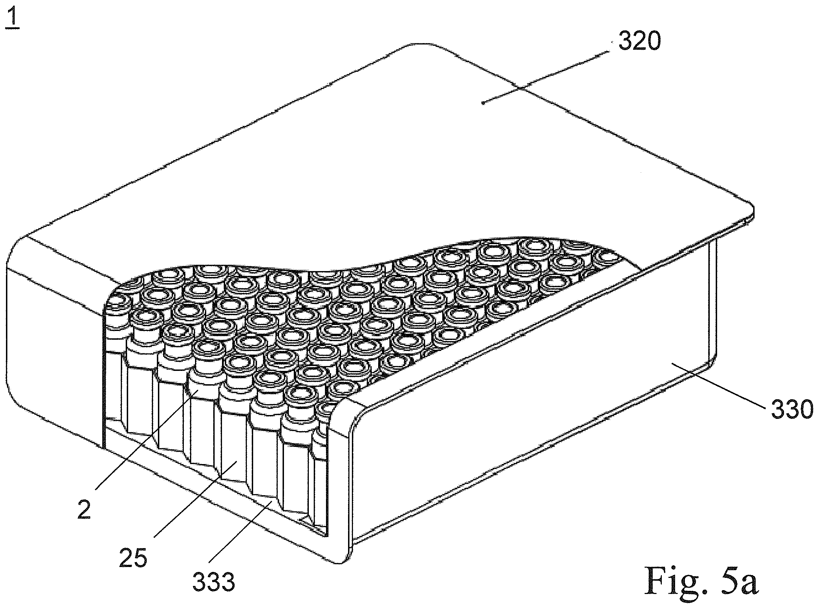

Unweigerlich weisen solche Behälter herstellungsbedingt Toleranzen auf, die gerade bei Glasfläschchen einen oder mehrere Zehntel Millimeter betragen können. Um solche Fertigungstoleranzen kompensieren zu können und gleichzeitig zu gewährleisten, dass sämtliche Böden 3 oder untere Enden der Behälter in einer Ebene angeordnet werden können, werden die Behälter in der Ausführungsform gemäß den Figuren 5a-5c mittels eines Reibschlusses bzw. einer Klemmung an einer Halterungsstruktur fixiert. Dieser Reibschluss wird entweder im Bereich der zylindrischen Seitenwand 4 oder am unteren, geschlossenen Ende bzw. Boden der Behälter oder gemäß weiteren bevorzugten Ausführungsformen im Bereich des verengten Halsabschnitts 5 realisiert. Im letztgenannten Fall wird jedenfalls die große Mehrzahl der Behälter reibschlüssig im Bereich des verengten Halsabschnitts 5 abgestützt, was jedoch gemäß weiteren Ausführungsformen nicht ausschließen soll, dass bei einzelnen Behältern mit großen Fertigungstoleranzen hinsichtlich ihrer axialen Länge der Übergangsbereich zwischen dem oberen Rand 6 und dem verengten Halsabschnitt 5 nicht auch ausnahmsweise formschlüssig hintergriffen oder abgestützt wird.

Zum gleichzeitigen Halten einer Mehrzahl von Behältern wird eine Haltestruktur 25 (im Stand der Technik auch als sog. "Nest" bezeichnet) bereitgestellt, die nachfolgend anhand der Fig. 5a noch genauer beschrieben wird und die aus einem Kunststoff ausgebildet ist, beispielsweise spritzgegossen ist. Die Haltestruktur 25 weist eine Mehrzahl von Aufnahmen 39 auf, die sich in Längsrichtung der aufzunehmenden Behälter erstrecken und miteinander verbunden sind. Bevorzugt sind die Aufnahmen 39 auch mechanisch miteinander gekoppelt. Die Seitenwände 45 der Aufnahmen 39 sind ausreichend flexibel oder aufweitbar, so dass die Behälter 2 von oben oder von unten her in die Aufnahmen 39 eingeführt werden können. Dadurch können eine Mehrzahl von Behältern 2 reibschlüssig fixiert bzw. geklemmt gehalten werden. Aufgrund der Elastizität der Seitenwände der Aufnahmen können auch Fertigungstoleranzen in axialer und/oder radialer Richtung der Behälter ausgeglichen werden, insbesondere bei Medikamentenbehältern aus Glas. Insbesondere können auch Behälter mit unterschiedlichen Durchmessern von ein- und derselben Haltestruktur 25 reibschlüssig fixiert werden.

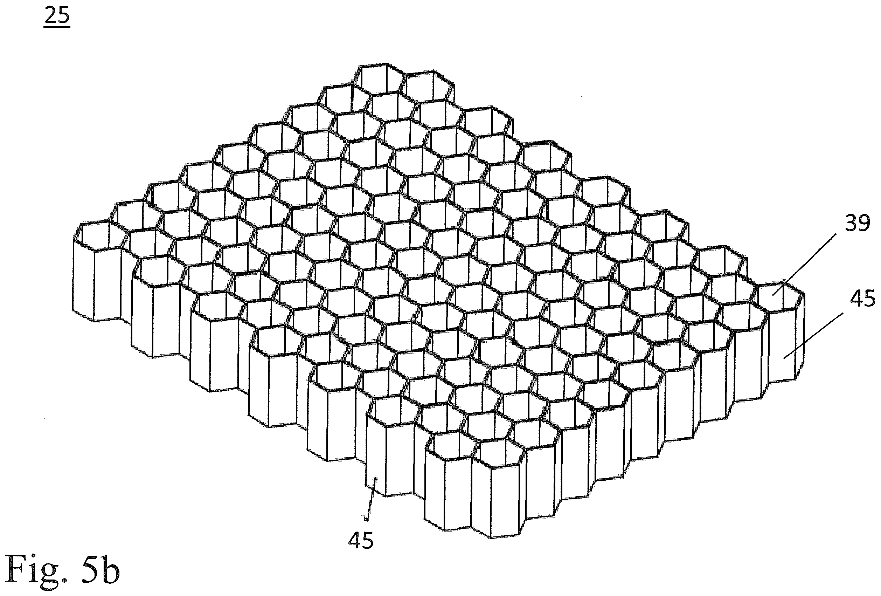

Wenn die Behälter in die Aufnahmen 39 einer Haltestruktur 25, wie in der Fig. 5b gezeigt, eingesetzt sind, können die unteren Enden der Behälter eine gewisse Strecke über den unteren Rand der Aufnahmen 39 hinausragen. Wenn der untere Bereich der Behälter durch Auflage auf einem Heizboden wärmebehandelt wird, führt dies aufgrund der vergleichsweise schlechten Wärmeleitungseigenschaften der Behälter (insbesondere aus Glas oder einem Kunststoff) zu einer verzögerten Erwärmung des Materials der Haltestruktur 25. Diese kann ausreichen, um eine Überhitzung der Haltestruktur auf Temperaturen oberhalb der Schmelz- oder Erweichungstemperatur ihres Materials zu verhindern.

Zum Transport und zur Verpackung einer Haltestruktur im Sinne der vorliegenden Anmeldung mit den darin aufgenommenen Behältern dient ein Transport- und Verpackungsbehälter 1 (im Stand der Technik auch als sog. "Tub" bezeichnet), wie schematisch in der Fig. 1c dargestellt. Gemäß der Fig. 1c ist der Transport- und Verpackungsbehälter 1 im Wesentlichen kasten- oder wannenförmig ausgebildet und weist ein unteres Segment 330 mit einem Boden 333 und einer Seitenwand und ein oberes Segment 320 auf, wobei die beiden Segmente 320, 330 in der Art von Schubfächern mit einem Boden und mit drei von diesem senkrecht abragenden Seitenwänden ausgebildet sind, die einander geschoben werden können, um den Transport- und Verpackungsbehälter 1 auszubilden, wie nachfolgend beschrieben.

Ein derartiger Transport- und Verpackungsbehälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Transport- und Verpackungsbehälter 10 aufgenommenen Haltestruktur 25 und der von dieser gehaltenen Behälter 2 zu ermöglichen.

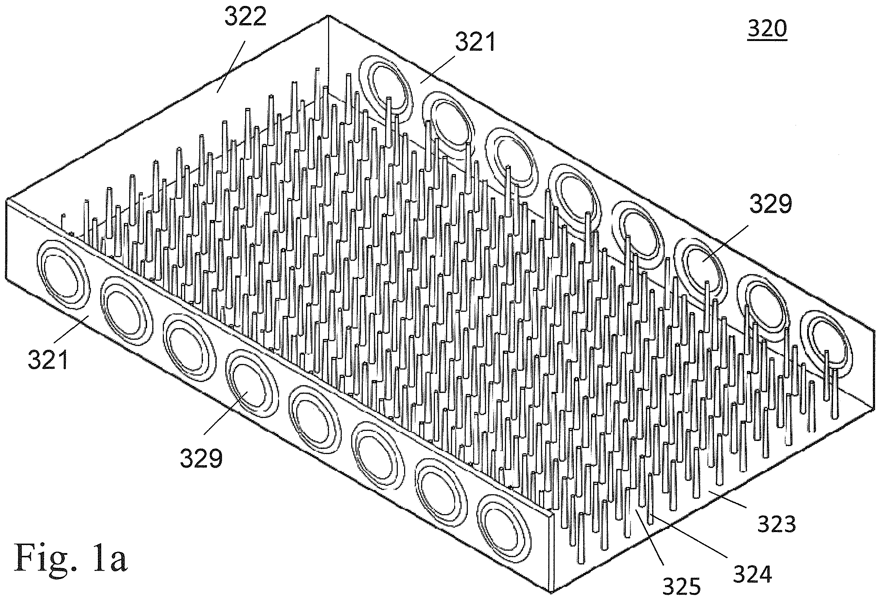

Anhand der Figuren 1a-1c wird nachfolgend ein Ausführungsbeispiel für einen Transport- und Verpackungsbehälter beschrieben, in dem die Behälter ohne zusätzliche Haltestruktur, also unmittelbar, aufgenommen sind. Die Fig. 1a zeigt ein oberes Segment 320, das in der Art eines Schubfaches mit einem Boden 323, zwei seitlichen Seitenwänden 321 und einer hinteren Seitenwand 322 ausgebildet ist, die beide rechtwinklig von dem Boden 323 abragen. In den Seitenwänden 321 sind runde Öffnungen 329 ausgebildet, die durch eine Schutzfolie, beispielsweise einem Geflecht aus Kunststofffasern, wie beispielsweise Polypropylen-Fasern (PP), oder einer Tyvek®-Schutzfolie steril verschlossen werden können.

Vom Boden 323 des oberen Segments 320 ragen eine Mehrzahl von Zapfen 324 rechtwinklig ab, die als Positionierungsmittel im Sinne der vorliegenden Anmeldung wirken, in einer regelmäßigen Anordnung angeordnet sind und jeweilige Aufnahmen 325 ausbilden, in denen die Behälter, beispielsweise Fläschchen (Vials), aufgenommen werden können (vgl. Fig. 1c).

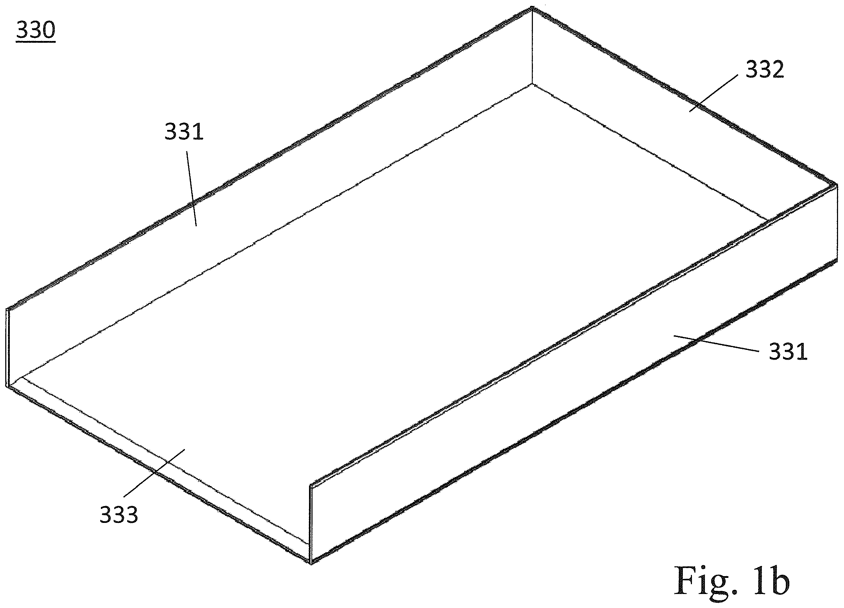

Die Fig. 1b zeigt ein dazu passend ausgebildetes unteres Segment 330, das ebenfalls in der Art eines Schubfaches mit einem Boden 333, zwei seitlichen Seitenwänden 331 und einer hinteren Seitenwand 332 ausgebildet ist, die beide rechtwinklig von dem Boden 333 abragen.

Die beiden Segmente 320, 330 können in der Art von Schubfächern ineinander geschoben werden, wie in der Fig. 1c dargestellt, um gemeinsam einen Transport- und Verpackungsbehälter 1 auszubilden, in dem die Behälter 2 in einer regelmäßigen Anordnung und abgedichtet gegen die Umgebung aufgenommen sind. Die Behälter 2 können aufrecht oder kopfüber in den von den Zapfen 324 ausgebildeten Aufnahmen angeordnet sein. Die Böden der Behälter 2 können dabei unmittelbar auf dem Boden 333 des oberen Segments abgestützt sein.

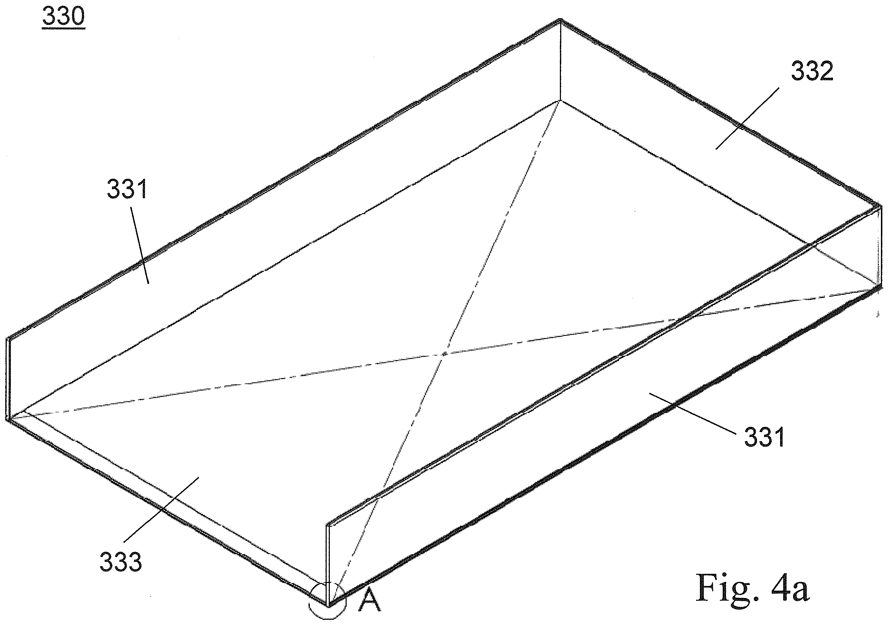

Die beiden Segmente 320, 330 können mit den von einem der beiden Segmente positionierten Behältern ineinander geschoben werden. Weitere Maßnahmen zur Abdichtung des Transport- und Verpackungsbehälters 1 werden nachfolgend anhand der Figuren 4a-4d beschrieben werden.

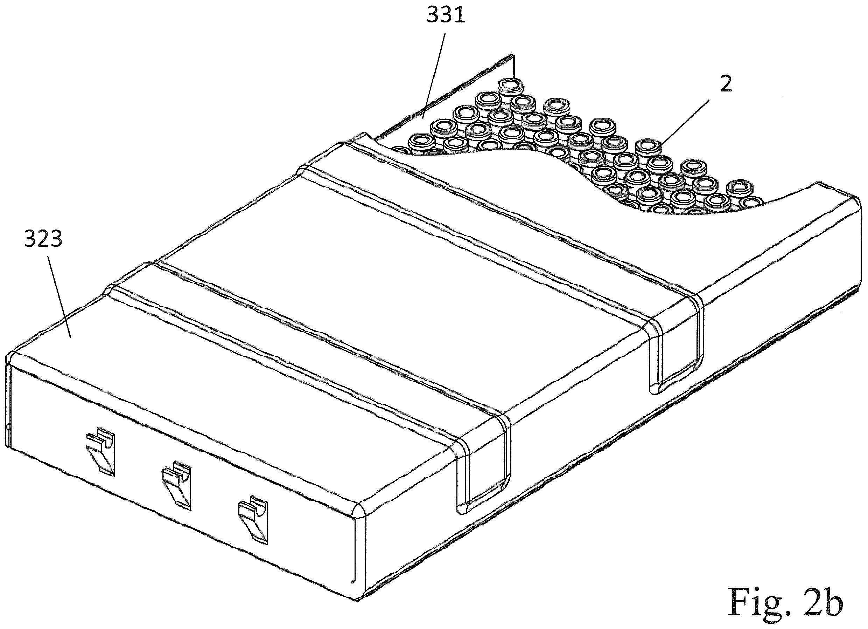

Die Fig. 2a und 2b zeigen eine weitere Ausführungsform für einen solchen Transport- und Verpackungsbehälter 1.

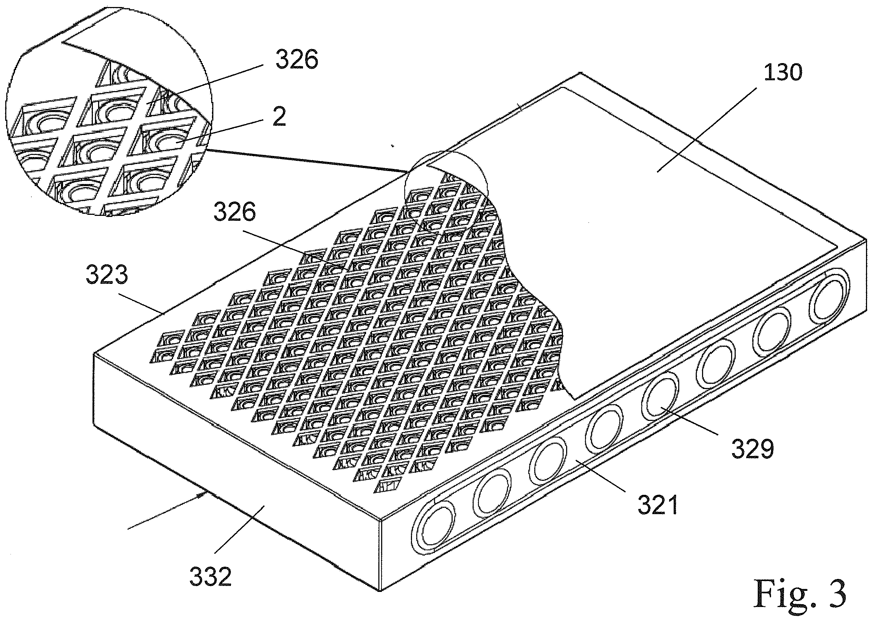

Die Fig. 3 zeigt eine weitere Ausführungsform für einen Transport- und Verpackungsbehälter 1, bei dem der Boden 323 des oberen Segments mit einer siebartigen Struktur ausgebildet ist, die von sich kreuzenden Kunststoffstegen 326 ausgebildet ist und Durchbrechungen ausbildet, durch die ein Gas zum Sterilisieren des Innenraums des Transport- und Verpackungsbehälters 1 einströmen kann. Der Transport- und Verpackungsbehälter 1 wird durch eine Schutzfolie 130 steril abgeschlossen, wie vorstehend beschrieben. Die Schutzfolie kann auch die kreisförmigen Öffnungen 329 abdecken.

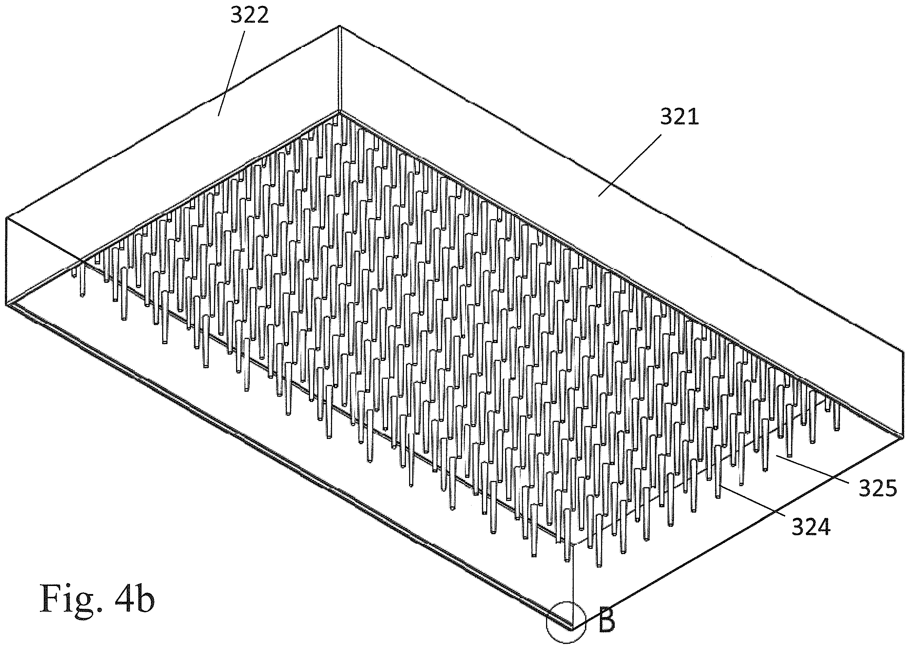

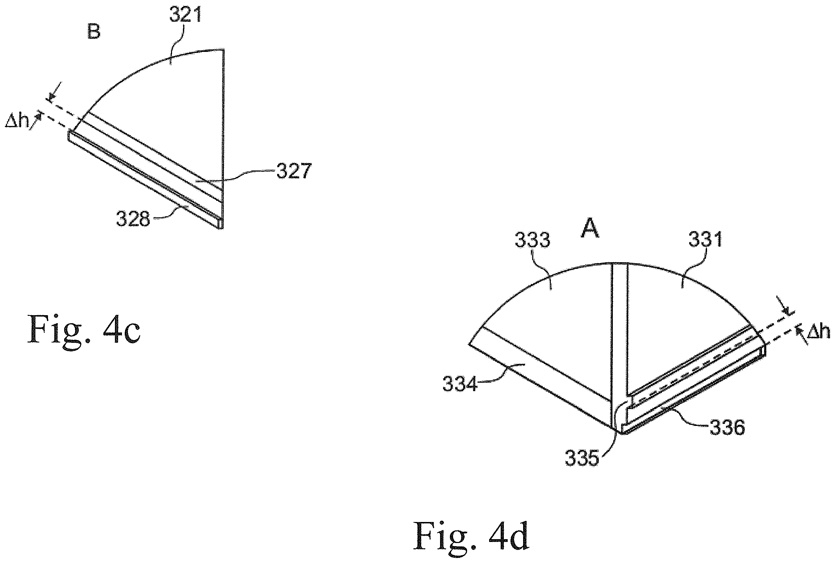

Die Figuren 4a bis 4d zeigen weitere Einzelheiten der beiden schubfachartigen Segmente 320, 330 des Transport- und Verpackungsbehälters. Gemäß der vergrößerten Darstellung des Bereichs A in der Fig. 4d ist auf der Seitenwand 331 des oberen Segments eine rechteckförmige Führungsschiene 335 ausgebildet, der sich in Längsrichtung erstreckt und als Vorsprung der zugeordneten Seitenwand 321 des unteren Segments 320 (vgl. Fig. 4b) zugewandt ist. Gemäß der vergrößerten Darstellung des Bereichs B in der Fig. 4c ist auf der Seitenwand 321 des unteren Segments eine korrespondierend rechteckförmig ausgebildete Ausnehmung 327 ausgebildet, die sich in Längsrichtung erstreckt, um die Führungsschiene 335 aufzunehmen und zu führen. Schon durch die Formgestaltung der Ausnehmung 327 und der Führungsschiene 335 wird eine Abdichtung des Innenraums des Transport- und Verpackungsbehälters erzielt.

Diese Abdichtung kann durch weitere Dichtungsmittel unterstützt werden, die entlang dem unteren der beiden Segmente ausgebildet sind, wie in den Fig. 4c und 4d schematisch dargestellt. Gemäß der Fig. 4c ist entlang dem unteren Rand der Seitenwand 321 des unteren Segments ein rechteckförmiger Vorsprung 328 ausgebildet, der sich in Längsrichtung erstreckt. Gemäß der Fig. 4d ist entlang dem unteren Rand der Seitenwand 331 des oberen Segments eine rechteckförmige Ausnehmung 336 ausgebildet, die sich in Längsrichtung erstreckt. Vorsprung 328 und Ausnehmung 336 sind korrespondierend zueinander ausgebildet und können beim Ineinanderschieben der beiden schubfachartigen Segmente in einen gegenseitigen Eingriff geraten, wodurch eine weitere Abdichtung in diesem Bereich erzielt wird.

Gemäß den Fig. 4c und 4d ist zwischen den Ausnehmungen und Vorsprüngen 327, 328 bzw. 336, 335 ein Abstand Δh ausgebildet, der jeweils gleich ist. Auf den Oberflächen der Ausnehmungen und Vorsprünge 327, 328 bzw. 336, 335 kann eine Kunststoffauflage oder Dichtungslippe (nicht gezeigt) angeordnet sein, um in diesem Bereich für eine weitere Abdichtung zu sorgen. Diese Abdichtfunktion kann durch elastische Eigenschaften einer solchen Kunststoffauflage oder Dichtungslippe weiter verstärkt werden.

Ähnliche Maßnahmen zur Abdichtung des Innenraums des Transport- und Verpackungsbehälters sind gemäß der Fig. 4a auch am vorderen Rand 333 des oberen Segments 330 vorgesehen. Zu diesem Zweck kann entlang dem vorderen Rand 333 eine Ausnehmung oder ein Vorsprung ausgebildet sein, der mit einem korrespondierend ausgebildeten Vorsprung oder einer korrespondierend ausgebildeten Ausnehmung zusammenwirkt, um für eine Abdichtung in diesem Bereich zu sorgen. Selbstverständlich kann auch in diesem Bereich eine zusätzliche Kunststoffauflage oder Dichtungslippe (nicht gezeigt) angeordnet sein, wie vorstehend beschrieben.

In den vorstehend beschriebenen Anordnungen sind die Behälter 2 in einer regelmäßigen Anordnung entlang von zwei zueinander orthogonalen Richtungen und in einer Ebene unter vorbestimmten konstanten Abständen zueinander verteilt angeordnet. Grundsätzlich sind auch weitere regelmäßige Anordnungen denkbar, beispielsweise können zueinander benachbarte Reihen bzw. Spalten von Behältern 2 auch um eine vorbestimmte Länge versetzt zueinander angeordnet sein, und zwar in einer wiederkehrenden Anordnung mit vorbestimmter Periodizität. Somit können automatisierte Fertigungsanlagen die Behälter 2 bei Übergabe an eine Bearbeitungsstation an präzise vorgebbaren Positionen erwarten, was den Automatisierungsaufwand erheblich verringert. Die Weiterverarbeitung der Behälter 2 kann erfolgen, während diese in einer Haltestruktur 25 aufgenommen sind und/oder während die Behälter in einem der Segmente des Transport- und Verpackungsbehälters aufgenommen sind.

Hierzu ist es zweckmäßig, wenn der Boden des Transport- und Verpackungsbehälters aus einem Metall ausgebildet ist, wobei das Metall zweckmäßig mit einem Kunststoff beschichtet ist, insbesondere einem thermoplastischen Kunststoff, der Temperaturen von bis zu 330°C und bevorzugter bis 350 °C resistiert, insbesondere Polyimide (PI) mit einem Schmelzpunkt von etwa 368°C, Polyetherketon (PEK) mit einem Schmelzpunkt von etwa 375°C, Polyetheretherketon (PEEK) mit einem Schmelzpunkt von etwa 341°C, Polyetherketonketon (PEKK) mit einem Schmelzpunkt von etwa 380°C oder Polyetherketonetherketonketon (PEKEEK) mit einem Schmelzpunkt von etwa 384°C.

Hierzu kann es auch zweckmäßig sein, wenn der Boden des Transport- und Verpackungsbehälters aus einem hochtemperaturfesten Kunststoff ausgebildet ist, insbesondere aus einem thermoplastischen Kunststoff oder aus Polyimdide (PI) oder aus Polyetherketon (PEK) oder aus Polysulfon (PSU) oder aus Polyetheretherketon (PEEK) oder bevorzugt aus einem Polyetheretherketon (PEEK) mit einem Schmelzpunkt oberhalb von 330 °C, bevorzugter bis zu 350°C.

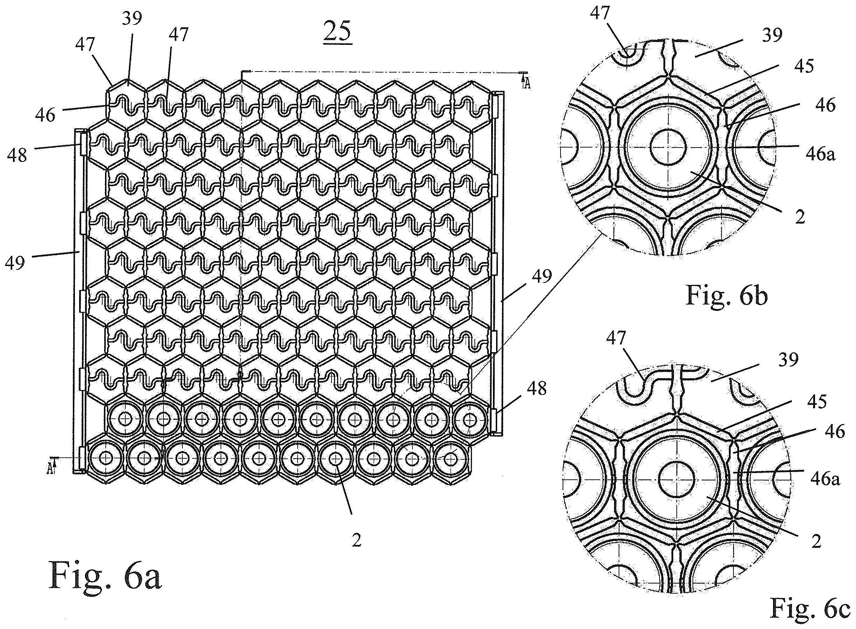

Die Figuren 6a bis 6e zeigen ein weiteres Ausführungsbeispiel für eine Haltestruktur (Träger). Diese ist von einer Mehrzahl von hexagonalen Aufnahmen 39 zur Aufnahme von Behälter 2 ausgebildet, die von zwei Paaren von unter einem stumpfen Winkel aufeinander zu laufenden Seitenwänden 45 ausgebildet werden, die über flexible Klemmstege 46 miteinander verbunden sind. Wie man der Fig. 6b entnehmen kann, weisen die flexiblen Klemmstege 46 einen jeweils konkav ausgebildeten Halteabschnitt 46a auf, wobei die Verbindungsstege 47 spiegelsymmetrisch ausgebildet sind, so dass die konkaven Einbauchungen auf den beiden Seiten der Klemmstege 46 den Seitenwänden der zugeordneten Behälter 2 zugewandt sind. Am unteren Ende der Halteaufnahmen 39 verbinden wellenlinienförmige Stege 47 die beiden Klemmstege 46a miteinander. Die Stege 47 können als Haltestege zur Abstützung der in den Aufnahmen 39 aufgenommenen Behältern 2 dienen. Alternativ können die Stege 47 jeweils einander gegenüberliegende Seitenwände der Aufnahmen 39 elastisch gegeneinander vorspannen, sodass ohne weiteres Behälter mit unterschiedlichen Durchmessern geklemmt werden können. Die Haltestruktur 25 gemäß der Fig. 6a kann grundsätzlich einstückig aus einem Kunststoff spritzgegossen sein.

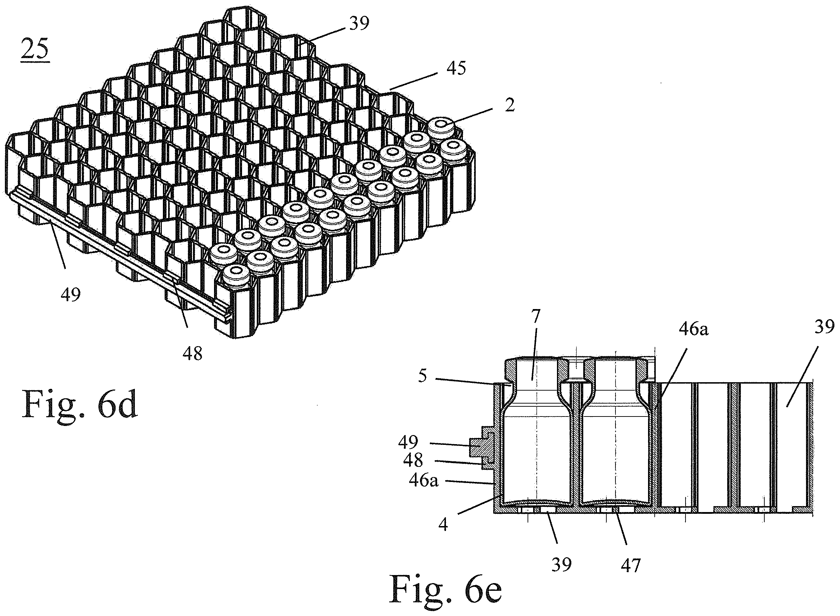

Die wellenlinienförmigen Stege 47 drücken die Aufnahmen 39 der Haltestruktur 25 in der Darstellung gemäß der Fig. 6a seitlich auseinander, so dass die Klemmstege 46 mit ihren Einbauchungen 46a in der in der Fig. 6c dargestellten ersten Stellung gegen die Seitenumfangswand der Behälter 2 gedrückt werden, sodass die Behälter 2 mit ausreichenden Haltekräften in den Aufnahmen 39 aufgenommen sind, wie in dem Einsatz gemäß der Fig. 6b dargestellt. Um diese Haltestruktur 25 ausgehend von der Stellung gemäß der Fig. 6b in die in der Fig. 6c dargestellte zweite Stellung zu überführen, in welcher die Klemmstege 46 mit ihren Einbauchungen 46a die Seitenumfangswände der Behälter 2 nicht mehr klemmen oder allenfalls nur noch mit einer geringen Kraft, sodass die Behälter aus den Aufnahmen ohne größeren Kraftaufwand entnommen bzw. darin verstellt werden können, muss die Haltestruktur 25 in der Darstellung gemäß der Fig. 6a in Querrichtung auseinandergezogen werden. Zu diesem Zweck sind an den Außenwänden der äußersten hexagonalen Aufnahmen 39 im Profil L-förmige Formschlusselemente 48 vorgesehen sind, die T-förmige Aufnahmen ausbilden, in die eine korrespondierend ausgebildete Rastschiene 49 eingeschoben ist. Durch Ziehen an diesen Rastschienen in Querrichtung kann die Haltestruktur insgesamt aufgespreizt werden, unter gleichzeitiger Aufweitung sämtlicher Aufnahmen 39. Je nachdem, wie stark seitlich an den Rastschienen 49 gezogen wird, können die Aufnahmen 39 geeignet aufgeweitet werden. Durch Stauchen der Halteaufnahmen 39 können somit insgesamt die Öffnungsweiten der Aufnahmen 39 koordiniert von der ersten Stellung gemäß der Fig. 6c in die zweite Stellung gemäß der Fig. 6b überführt werden, in welcher die Behälter 2 an vorbestimmten Positionen reibschlüssig fixiert sind. Die Höhenlage der Behälter 2 wird dabei aufgrund der Auflage der Böden der Behälter 2 auf den Stegen 47 im Wesentlichen durch die Stege 47 festgelegt. Wie man der Schnittdarstellung gemäß der Fig. 6e entnehmen kann, ist auch bei dieser Ausführungsform der größte Teil des Bodens der Behälter 2 von unten her frei zugänglich, beispielsweise für eine mechanische Verstelleinrichtung.

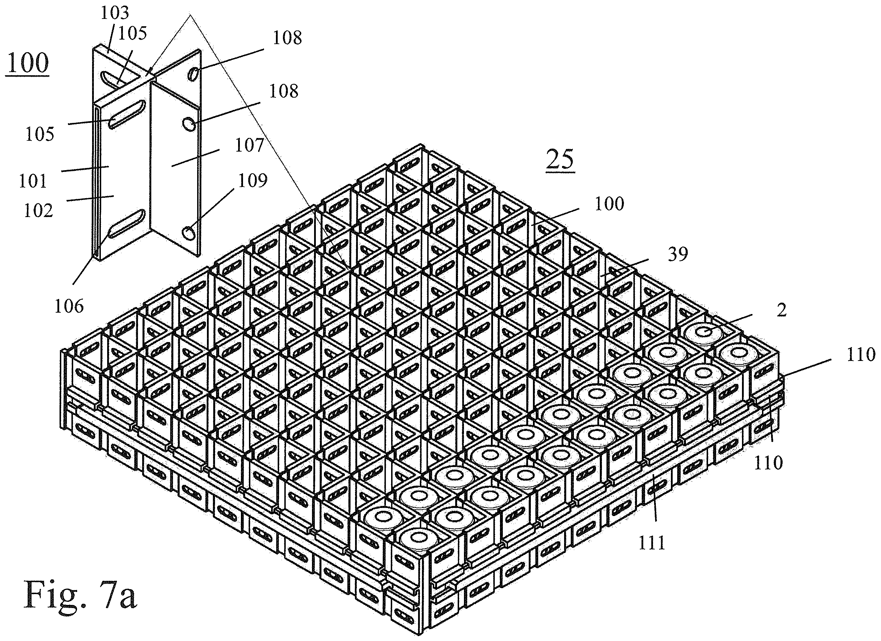

Die Figuren 7a - 7c zeigen ein weiteres Beispiel für eine Haltestruktur zur Verwendung in einem Transport- und Verpackungsbehälter gemäß der vorliegenden Erfindung. Gemäß dem oberen Bildteil der Fig. 7a weist weist diese sich kreuzförmig schneidende Seitenwände 101, 103, 107 mit identischen Schenkellängen auf. Zwei der Seitenwände, nämlich die Seitenwände 101, 103 sind geschlitzt ausgebildet, mit einem Längsschlitz 102, der sich im Wesentlichen über die gesamte Höhe der Seitenwände 101, 103 erstreckt. In den Seitenwänden 101, 103 sind ein oberes Langloch 105 und ein unteres, parallel dazu verlaufendes Langloch 106 ausgebildet. Die Dicke der Seitenwände 107 ist auf die Breite des Längsschlitzes 102 abgestimmt, so dass diese darin aufgenommen werden können. Auf den Seitenwänden 107 sind zylindrische Vorsprünge 108, 109 ausgebildet, die, wenn die Seitenwände 107 in dem zugeordneten Längsschnitz 102 eingeführt sind, in dem oberen bzw. unteren Langloch 105, 106 geführt sind. Eine Mehrzahl solcher Grundeinheiten 100 wird zu einer rechteckförmigen Haltestruktur 25 zusammengesteckt, wie im rechten unteren Bildteil der Fig. 7a dargestellt.

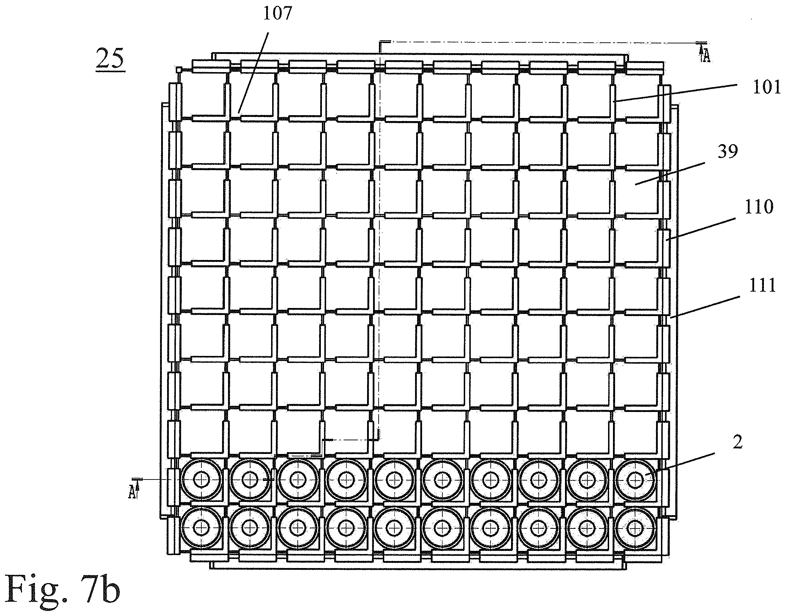

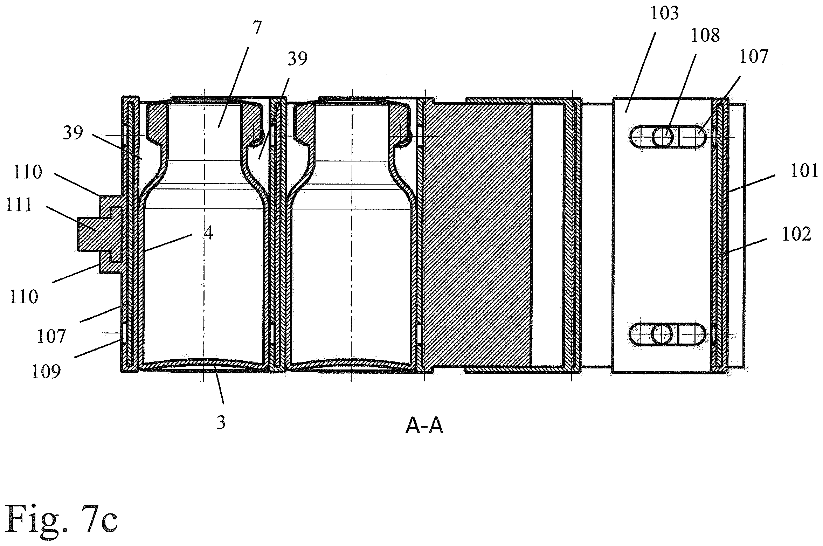

Die Fig. 7b zeigt die Haltestruktur 25 gemäß der Fig. 7a in einer schematischen Draufsicht. Die Seitenwände 101 der Grundeinheiten dienen als Führungsplatten für die darin aufgenommenen Rastplatten 107 der Grundeinheiten. Aufgrund des Zusammenwirkens der Vorsprünge 108, 109 mit den zugeordneten Langlöchern 105, 106 sind die Grundeinheiten in der Ebene der Haltestruktur 25 verschieblich gelagert. Durch das Zusammenwirken der Grundeinheiten werden somit eine Mehrzahl von im Wesentlichen quadratischen oder rechteckförmigen, länglichen Aufnahmen 39 ausgebildet, in die die Behälter 2 von oben oder von unten her eingeführt werden können. Zum Einführen der Behälter werden die Grundeinheiten so auseinandergespreizt, dass die Öffnungsweiten der Aufnahmen 39 ein ungehindertes Einführen oder jedenfalls ein Einführen der Behälter mit nur geringem Kraftaufwand ermöglichen. Anschließend können sämtliche Grundeinheiten so koordiniert gegeneinander gedrückt werden, dass die in den Aufnahmen 39 aufgenommenen Behälter 2 schließlich mit ausreichender Kraft reibschlüssig fixiert, insbesondere geklemmt werden. Die Fig. 7c zeigt die Haltestruktur 25 gemäß der Fig. 7b in einem Teil-Längsschnitt entlang der Linie A-A gemäß der Fig. 7b.

Die rechteckförmige Grundform der Haltestruktur 25 wird bei diesem Ausführungsbeispiel durch im Profil L-förmige Formschlusselemente 110 bewerkstelligt, die auf den Außenwänden der äußeren Grundeinheiten angeordnet sind, so dass eine im Wesentlichen U-förmige Aufnahme mit einem Längsschlitz in der Basis ausgebildet ist, in welchen die mit einem korrespondierenden T-förmigen Profil versehene Rastschiene 111 eingreift. Mittels der Rastschiene 111 kann auch eine Fixierung der Position sämtlicher Grundeinheiten 100 erzielt werden, beispielsweise durch Festschrauben oder Festklemmen der Rastschiene 111 jedenfalls an den vordersten und hintersten Grundeinheiten 100 einer Spalte bzw. Reihe der matrixförmigen Haltestruktur 25.

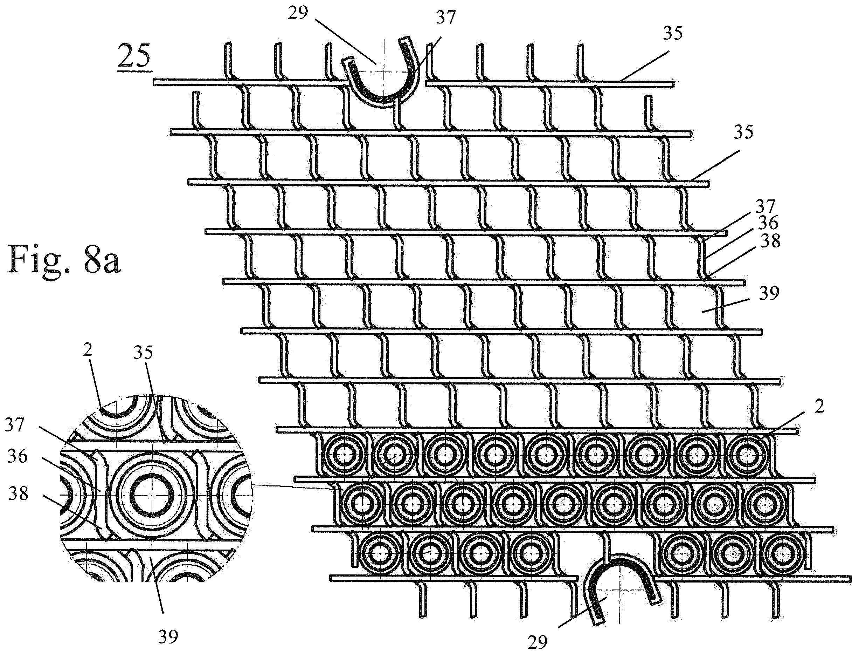

Die Figuren 8a - 8d zeigen ein weiteres Beispiel für eine Haltestruktur zur Verwendung in einem Transport- und Verpackungsbehälter gemäß der vorliegenden Erfindung. Diese weist eine Mehrzahl von parallel zueinander verlaufenden Querstegen 35 auf, die über unter regelmäßigen Abständen zueinander angeordnete S-förmige Verbindungsstege 36 miteinander verbunden sind, die im Wesentlichen rechtwinklig zu den Querstegen 35 verlaufen. Genauer gesagt sind die Verbindungsstege 36 über in entgegengesetzte Richtungen gekrümmte vordere bzw. hintere Enden 37, 38 mit den Querstegen 35 verbunden. Die Verbindungsstege 36 sind aus einem Kunststoff hergestellt, bevorzugt aus einem flexiblen Kunststoff. Die Querstege 35 weisen bevorzugt eine größere Steifigkeit als die Verbindungsstege 36 auf. Aufgrund der S-förmigen Formgebung der Verbindungsstege 36 sind die Querstege 35 jeweils zueinander in deren Längsrichtung um eine konstante Distanz versetzt, so dass die Haltestruktur 25 insgesamt als Parallelogramm ausgebildet ist, mit einer Basis im Bereich des unteren Rands der in der Fig. 8a dargestellten Haltestruktur 25 und zwei dazu unter einem spitzen Winkel geneigt verlaufenden imaginären Randlinien, welche die vorderen Enden der Querstege 35 miteinander verbinden. In der im rechten Bildteil der Fig. 8a dargestellten entspannten Ausgangsposition können die Behälter 2 frei und ohne Berührung mit den Stegen 35, 36 oder jedenfalls mit nur geringem Kraftaufwand in die von den Stegen 35,36 ausgebildeten länglichen Halteaufnahmen 39 eingeführt werden. Die Halteaufnahmen 39 weisen im Wesentlichen einen quadratischen Querschnitt auf, der so auf den Durchmesser der Behälter 2 abgestimmt ist, dass diese darin in einer zweiten Stellung der Haltestruktur 25 mit ausreichender Kraft reibschlüssig fixiert, insbesondere geklemmt werden können.

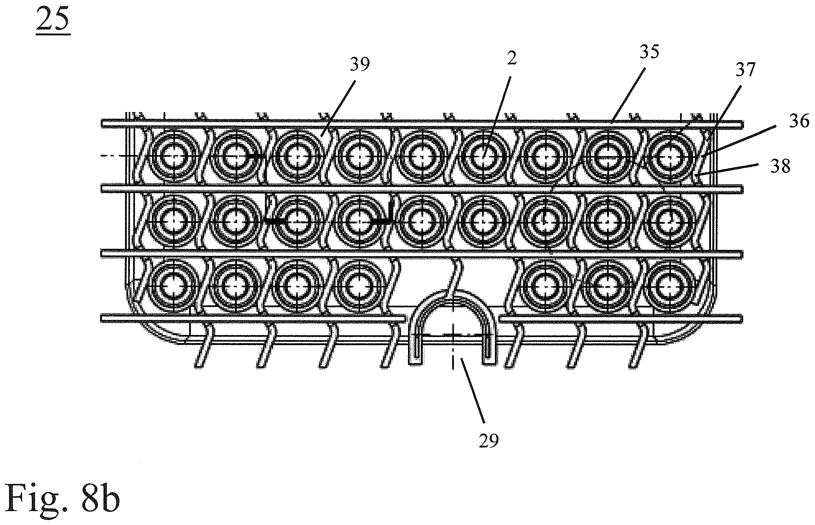

Um die Haltestruktur 25 aus der in der Fig. 8a dargestellten ersten Stellung in die zweite Stellung zu überführen, die in der Fig. 8b dargestellt ist, werden die Querstege 35 jeweils in deren Längsrichtung so verschoben, dass schließlich die in der Fig. 8b dargestellte rechteckförmige oder quadratische Haltestruktur 25 ausgebildet ist. Wie dem Vergleich der Fig. 8a und 8b entnommen werden kann, verbiegen sich hierzu die Verbindungsstege 36 geringfügig. Dabei werden sämtliche Seitenwände der Aufnahmen 39 beim Verschieben der Querstege 35 koordiniert, d.h. gemeinsam, von der ersten Stellung in die zweite Stellung verstellt, und zwar durch Verschwenken des oberen Endes der Haltestruktur 25 (vgl. Fig. 8a) relativ zu der Basis am unteren Ende des in der Fig. 8a dargestellten Parallelogramms.

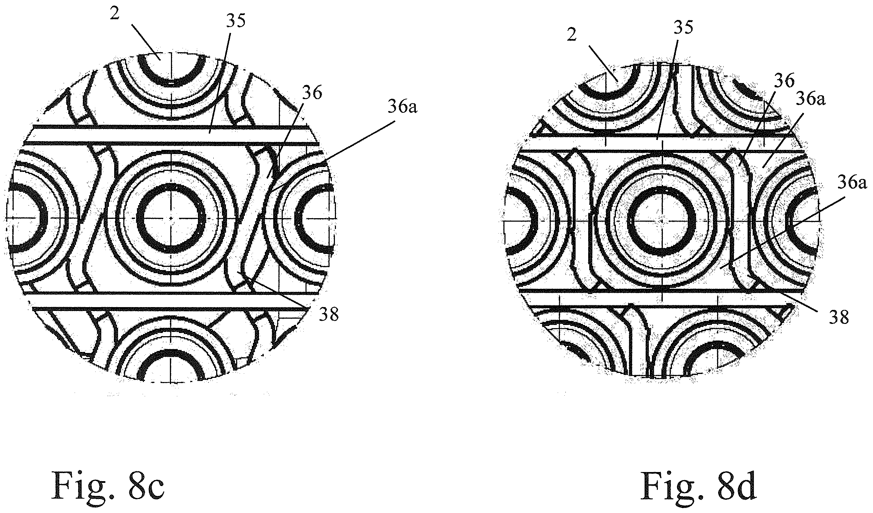

Die Figuren 8c und 8d zeigen in stark vergrößerten Teildarstellungen die Fixierung der Behälter 2 an einer solchen Haltestruktur. An den Verbindungsstegen 36 auf den beiden Seiten ist jeweils ein konkav ausgebildeter Abschnitt 36a ausgebildet, wobei der Krümmungsradius der beiden konkaven Aufnahmen der Abschnitte 36a auf den Radius der Behälter 2 abgestimmt ist. In der zweiten Stellung gemäß der Fig. 8c, in welcher die Verbindungsstege 36 sich geneigt zu den Querstegen 35 erstrecken, schmiegen sich die konkaven Aufnahmen 36a an die zylindrische Seitenwand der Behälter 2 an, sodass die Behälter noch zuverlässiger und kontrollierter gehalten werden können. In der ersten Stellung gemäß der Fig. 8d, in welcher die Verbindungsstege 36 sich senkrecht zu den Querstegen 35 erstrecken, sind die konkaven Aufnahmen 36a nicht mehr gegenüberliegend zur zylindrischen Seitenwand der Behälter 2 angeordnet, sodass die Behälter ungehindert, jedenfalls mit deutlich reduziertem Kraftaufwand, in die von den Stegen 35, 36 ausgebildeten Aufnahmen eingeführt und aus diesen herausgenommen werden können. Idealerweise liegen die Stege 35, 36 in der ersten Stellung gemäß der Fig. 8d überhaupt nicht an den Seitenwänden der Behälter 2 an.

Während bei den vorherigen Ausführungsbeispielen dargelegt wurde, dass die Behälter aufrecht und mit ihrem offenen Ende zum oberen Ende des Transport- und Verpackungsbehälters hin angeordnet sind, können die Behälter grundsätzlich auch gewendet, d.h. mit ihrem offenen Ende hin zum Boden des Transport- und Verpackungsbehälters zeigend, angeordnet sein.

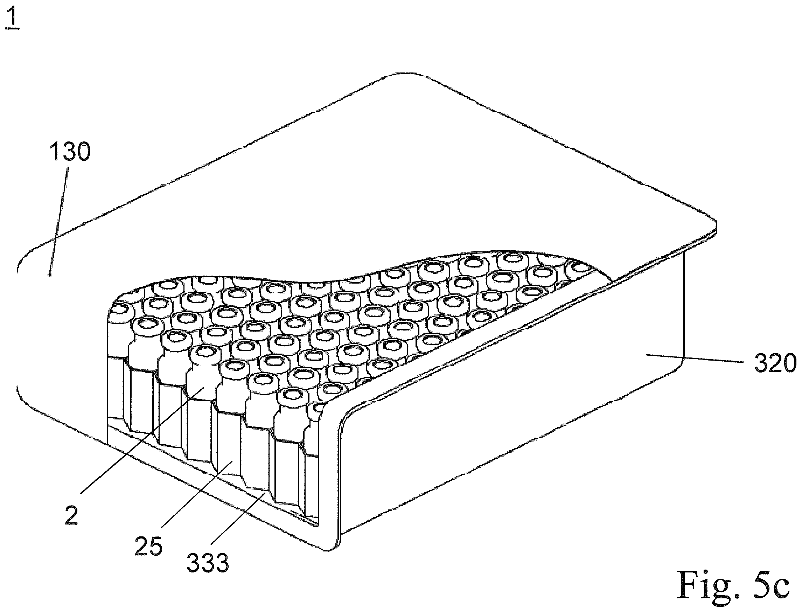

Die Oberseite oder die Ober- und Unterseite einer Haltestruktur 25 gemäß der vorliegenden Erfindung oder auch eines Transport- und Verpackungsbehälters 1 gemäß der vorliegenden Erfindung kann bzw. können von einer sterilen, gasdurchlässigen Schutzfolie überzogen sein, die aufgeklebt und bei Bedarf abziehbar ist. Dies ist beispielhaft in der Fig. 5c dargestellt, wonach die Folie 130 auf den oberen Rand der unteren Segments 320 aufgeklebt ist. Bei der Schutzfolie kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 mittels eines Gases ermöglicht, das durch die Folie 130 hindurch einströmt.

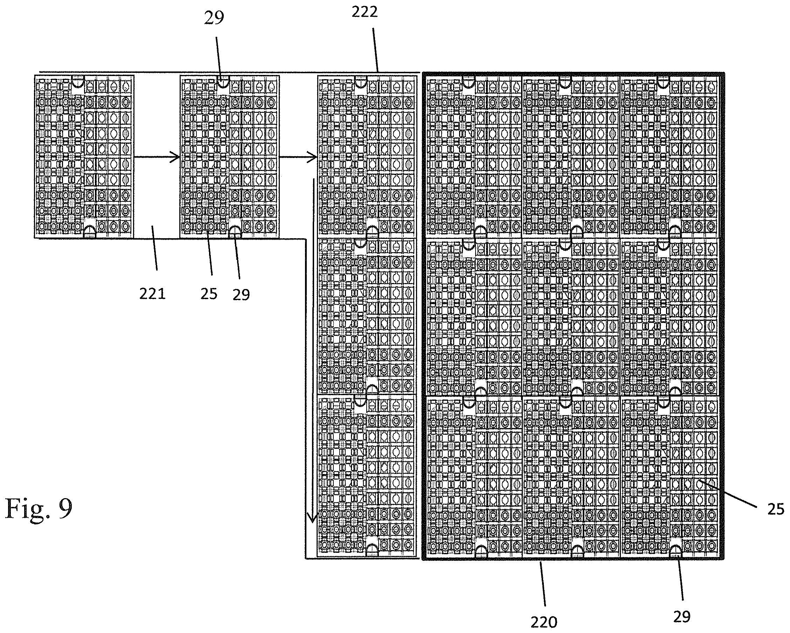

Die Fig. 9 zeigt schematisch in einer Draufsicht ein Verfahren, bei dem eine Mehrzahl von Behältern, während diese in eine Träger 25, wie vorstehend beschrieben, in einer regelmäßigen Anordnung gehalten werden, bei sehr hohen Temperaturen prozessiert werden, beispielsweise in einer Wärme-Prozessierungsstation, insbesondere in einem Heißofen oder einem Heißtunnel, mit Temperaturen von bis zu 330°C und bevorzugter bis zu 350°C.

Gemäß der Fig. 9 werden die Träger 25 mit den von diesen in einer regelmäßigen zweidimensionalen Anordnung gehaltenen Behältern mittels der Fördereinrichtung 221, beispielsweise eines Transportbands oder einer Rollenbahn, in Pfeilrichtung in Richtung zu einem Heißofen oder Heißtunnel 220 gefördert. Dieser kann beispielsweise seitlich zu einer Haupt-Fördereinrichtung einer nicht dargestellten Prozessanlage angeordnet sein, von der die Träger 25 auf die Fördereinrichtung 221 umgesetzt bzw. umgelenkt und in Richtung zu einem Heißofen oder Heißtunnel 220 gefördert werden. Vor dem Heißofen oder Heißtunnel 220 befindet sich eine quer zur Fördereinrichtung 221 verlaufende Ablagefläche, auf der die Träger gesammelt werden. Diese Sammlung der Träger 25 vor dem Heißofen oder Heißtunnel 220 kann auch auf mehreren Ebenen erfolgen, in Entsprechung zu den Ebenen des Heißofens oder Heißtunnels 220.

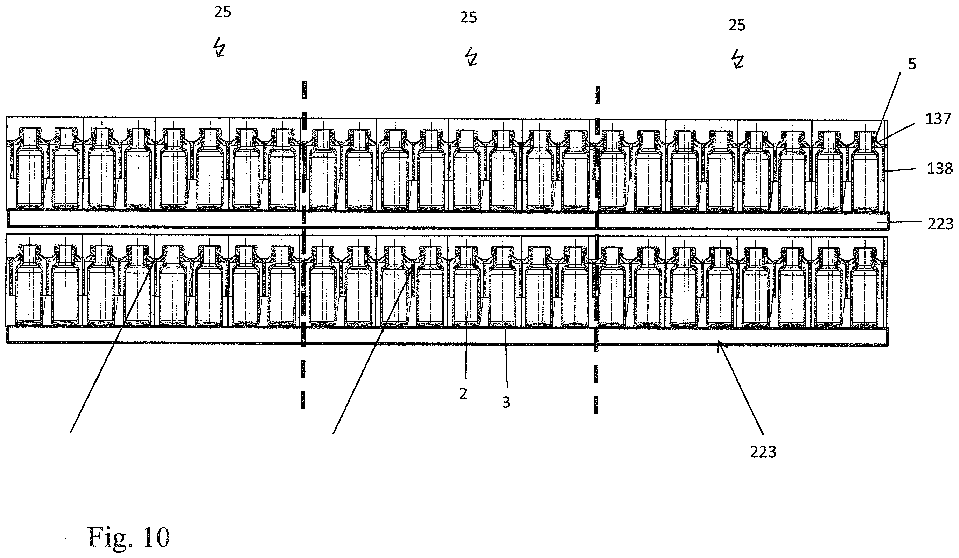

Um die Grundfläche der Träger 25 weiter zu reduzieren, kann es hierfür von Vorteil sein, wenn Randabschnitte 150 der Träger 25 abgenommen oder weggeschwenkt werden können. Diese einfache Maßnahme erhöht die erzielbare Packungsdichte der Behälter 2 beim Beladen des Heißofens oder Heißtunnels 220 weiter. Die Fig. 10 zeigt einen vergrößerten Teilschnitt durch einen Gefriertrockenschrank. Wie man erkennen kann, liegen die Böden 3 der Behälter 2 unmittelbar auf den auf sehr hohe Temperaturen befindlichen Böden 223 auf, sodass eine optimale und rasche Erwärmung der Behälter 2 erzielt werden kann, insbesondere ihrer Böden und unteren Bereiche. Die Böden 223 sind dabei auf mehreren Etagen angeordnet.

Zur Prozessierung der Behälter 2 bei den sehr hohen Temperaturen müssen diese nicht aus den Trägern umständlich entnommen und vereinzelt werden. Vielmehr sind diese auch weiterhin während der Prozessierung zuverlässig in den Aufnahmen der Träger 25 aufgenommen. Um die Temperaturbeständigkeit der Träger 25 zu gewährleisten, ist der Träger 25 und/oder zumindest die Aufnahmen des Trägers 25 aus einem hochtemperaturfesten Kunststoff ausgebildet, insbesondere aus einem thermoplastischen Kunststoff, der den hohen Prozessierungstemperaturen von bis zu 330°C und bevorzugter bis zu 350°C widerstehen kann. Bevorzugt handelt es sich bei dem thermoplastischen Kunststoff um Polyimide (PI), Polyetherketon (PEK), Polyetheretherketon (PEEK), Polyetherketonketon (PEKK) oder Polyetherketonetherketonketon (PEKEEK) mit einem Schmelzpunkt von bis zu 330°C und bevorzugter bis zu 350°C.

Die Prozessierung der Mehrzahl von Behältern 2 in dem Heißofen oder Heißtunnel mit Temperaturen von bis zu 330°C und bevorzugter bis zu 350°C erfolgt derart, dass Endotoxine und/oder die Lyse von Bakterien und/oder Cytokine induzierende Substanzen in den Behältern 2 thermisch entfernt werden können.

Die Prozessierung der Mehrzahl von Behältern 2 in dem Heißofen oder Heißtunnel mit Temperaturen von bis zu 330°C und bevorzugter bis zu 350°C kann auch derart erfolgen, dass Silikonschichten auf Innenoberflächen der Behälter 2, insbesondere auf einem Boden 3 und unterem Bereich derselben, eingebrannt werden. Die Silikonisierung geschieht durch Aufbringen einer Menge von Silikonöl oder -emulsion auf zumindest einen Teilbereich der inneren Oberfläche jedes Behälters. Es kann hierbei eine vorbestimmte Menge an Silikon aufgebracht werden. Zusätzlich kann vorgesehen sein, überschüssiges Silikon sofort zu entfernen, beispielsweise durch Abwischen oder Spülen. Das aufgebrachte Silikonöl wird anschließend durch Wärmebehandlung bei Temperaturen zwischen 150°C und 350°C fixiert oder eingebrannt. Hierbei kann weiter vorgesehen sein, dass nicht gebundenes oder nicht kovalent bzw. nebenvalent gebundenes Silikon ganz oder teilweise durch Wischen oder Spülen entfernt wird.

Die vorgenannte Prozessierung der Behälter schließt selbstverständlich nicht aus, dass diese beispielsweise vorübergehend durch Anheben in eine andere Höhenposition verschoben werden. Dies kann beispielsweise durch Koordiniertes Verstellen von sämtlichen Aufnahmen 39 des Trägers 25 realisiert werden, wie beispielsweise in der Fig. 8a dargestellt. In dieser Stellung können die Behälter 2, während diese auch weiterhin in den Aufnahmen 39 des Trägers angeordnet oder zumindest darin geführt sind, in ihrer Höhenlage verstellt werden.

Gemäß einer alternativen Ausführungsform (nicht dargestellt) werden die Behälter in dem Heißofen oder Heißtunnel bei den sehr hohen Temperaturen von bis zu 330°C und bevorzugter bis zu 350°C prozessiert, während diese in einem Transport- und Verpackungsbehälter aufgenommen sind, wie vorstehend beschrieben, und von dessen Positionierungsmitteln zuverlässig in der regelmäßigen Anordnung positioniert sind. Um die Temperaturbeständigkeit des Transport- und Verpackungsbehälters und dessen Positionierungsmittel zu gewährleisten, ist der Transport- und Verpackungsbehälter oder zumindest dessen Bodenbereich, der von einem Segment, wie vorstehend beschrieben, ausgebildet sein kann, aus einem hochtemperaturfesten Kunststoff ausgebildet, insbesondere aus einem thermoplastischen Kunststoff, der Temperaturen von bis zu 330°C und bevorzugter bis 350 °C resistiert, insbesondere aus Polyimide (PI), Polyetherketon (PEK), Polyetheretherketon (PEEK), Polyetherketonketon (PEKK) oder Polyetherketonetherketonketon (PEKEEK).

Gemäß einer alternativen Ausführungsform ist der Transport- und Verpackungsbehälter oder zumindest dessen Bodenbereich aus einem Metall ausgebildet, das den Temperaturen bei der Prozessierung von bis zu 330°C und bevorzugter bis zu 350°C widerstehen kann.

Zur Prozessierung kann dabei zunächst der Transport- und Verpackungsbehälter, wie vorstehend beschrieben, durch Verschieben oder Freigeben von zumindest einem Segment der Mehrzahl von Segmenten 320, 330 (vgl. Fig. 1c, 2a oder 3) oder des Transport- und Verpackungsbehälters derart geöffnet werden, dass der Innenraum des Transport- und Verpackungsbehälters zugänglich ist, beispielsweise um ein Gas in den Innenraum einströmen zu lassen.

In diesem geöffneten Zustand oder alternativ, während der Transport- und Verpackungsbehälter geschlossen ist, erfolgt die Prozessierung in dem Heißofen oder Heißtunnel bei den sehr hohen Temperaturen von bis zu 330°C und bevorzugter bis zu 350°C, während die Behälter von den Positionierungsmitteln oder einem in den Transport- und Verpackungsbehälter angeordneten Träger in einer regelmäßigen Anordnung positioniert sind.

Sofern der Transport- und Verpackungsbehälter zur Prozessierung geöffnet wurde, erfolgt abschließend ein Zusammensetzen oder Zusammenstecken der Segmente, um den Transport- und Verpackungsbehälter erneut zu schließen, sodass die Dichtungsmittel den Innenraum des Transport- und Verpackungsbehälters gegen die Umgebung steril abdichten.

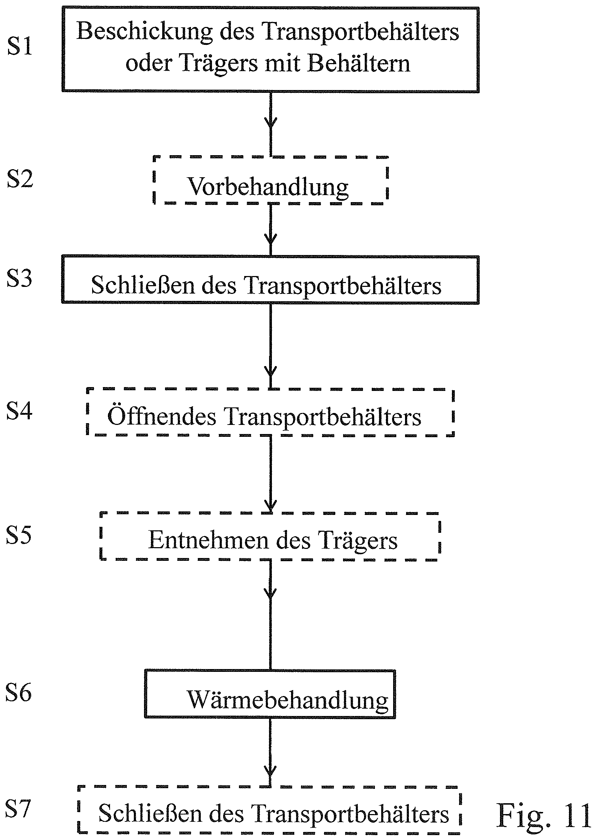

Anhand des schematischen Flussdiagramms gemäß der Fig. 11 soll beispielhaft ein Verfahren gemäß der vorliegenden Erfindung beschrieben werden. In der Fig. 11 bezeichnen dabei die mit gestrichelten Linien umrandeten Blöcke Verfahrensschritte, die optional ausgeführt werden können, was von der konkreten Ausführungsform abhängt.

In dem Schritt S1 wird der Transport- und Verpackungsbehälter (in der Fig. 11 vereinfachend als Transportbehälter bezeichnet) mit Behältern beschickt bzw. beladen, beispielsweise durch Einsetzen der Behälter in die von den Positionierungsmitteln 324 gemäß der Fig. 1a ausgebildeten Aufnahmen 325, oder der Träger mit Behältern beschickt bzw. beladen, beispielsweise mit Fläschchen oder Karpulen, wie beispielhaft in der Fig. 6d gezeigt. Anschließend kann in dem Schritt S2 eine Vorbehandlung des Transportbehälters erfolgen, beispielsweise ein Sterilisieren des Transportbehälters. Im Schritt S3 wird der Transportbehälter, in welchem die Behälter entweder unmittelbar aufgenommen sind oder in welchem ein Träger aufgenommen ist, in welchem seinerseits die Behälter aufgenommen sind, geschlossen, wie vorstehend beschrieben. In diesem Zustand sind die Behälter in dem Transportbehälter steril und zuverlässig positioniert aufgenommen und können beispielsweise aufbewahrt oder zu einem Pharmahersteller transportiert werden.

In dem optionalen Schritt S4 kann der Transportbehälter wieder geöffnet werden, um einen Zugriff auf die darin aufgenommenen Behälter oder den darin aufgenommenen Träger zu verschaffen. Sofern die Wärmebehandlung der Behälter nicht unmittelbar in dem Transportbehälter erfolgen soll, schließt sich der optionale Verfahrensschritt S5 an, in dem der Träger aus dem Transportbehälter entnommen wird.

In dem Verfahrensschritt S6 erfolgt anschließend eine Wärmebehandlung der Behälter, entweder während diese in dem bevorzugt geschlossenen Transportbehälter aufgenommen sind oder während diese in dem Träger nach dessen Entnahme aus den Transportbehälter aufgenommen sind. Die Wärmebehandlung kann insbesondere bei sehr hohen Temperaturen von bis zu 330°C und bevorzugter bis zu 350°C erfolgen.

Anschließend können die Behälter entweder unmittelbar weiter verarbeitet werden, beispielsweise befüllt und anschließend steril verschlossen und abgepackt werden, oder diese werden gemeinsam mit dem Träger wiederum in den Transportbehälter eingesetzt, der dann in dem optionalen Verfahrensschritt S7 erneut verschlossen wird, wie vorstehend beschrieben. In dem letztgenannten Zustand können die wärmebehandelten Behälter dann in dem Transportbehälter steril aufbewahrt oder transportiert werden.

Wie dem Fachmann beim Studium der vorstehenden Beschreibung ohne Weiteres sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Transport- und Verpackungsbehälter zum Aufnehmen einer Mehrzahl von zylindrischen Behältern (2) für Substanzen für medizinische, pharmazeutische oder kosmetische Anwendungen, mit zumindest zwei Segmenten (320, 330), die jeweils separat handhabbar und gemeinsam zu dem Transport-und Verpackungsbehälter (1) zusammensetzbar oder zusammensteckbar sind, wobei
ein erstes Segment (330; 320) der zumindest zwei Segmente einen Boden (333; 323) zum Abstützen der Mehrzahl von Behältern (2) aufweist, und
Positionierungsmittel (324; 25) vorgesehen sind, um die Mehrzahl von Behältern (2) im Innenraum des Transport und Verpackungsbehälters (1) in einer regelmäßigen Anordnung so zu positionieren, dass eine Berührung von unmittelbar benachbarten Behältern (2) verhindert ist, wobei
an den Segmenten (320, 330) elastische Dichtungsmittel (327, 328, 334, 335) vorgesehen sind, sodass die Segmente (320, 330) mehrfach zu dem Transport- und Verpackungsbehälter zusammensetzbar oder zusammensteckbar sind und der Innenraum des Transport- und Verpackungsbehälters (1) gegen die Umgebung abgedichtet ist,
**dadurch gekennzeichnet, dass** an den Segmenten korrespondierend zueinander ausgebildete Führungsschienen (327) und Führungsausnehmungen (335) vorgesehen sind, welche die Segmente beim Ineinanderschieben zum Zusammensetzen des Transport- und Verpackungsbehälters (1) führen, wobei
die Führungsschienen (327) oder Führungsausnehmungen (335) mit Dichtungslippen umgeben sind, die ein Eindringen von Verunreinigungen in den Innenraum des Transport- und Verpackungsbehälters verhindern, oder
die Führungsschienen (327) oder Führungsausnehmungen (335) mit einer elastischen Kunststoffauflage versehen sind, insbesondere ausgebildet in einer 2K-Spritzgusstechnik, sodass die elastische Kunststoffauflage elastisch gegen gegenüberliegende Seitenwände der Führungsschienen oder Führungsausnehmungen des anderen Segment drückt, um den Innenraum des Transport- und Verpackungsbehälters gezielt auch in diesen Bereichen abzudichten.

2. Transport- und Verpackungsbehälter nach Anspruch 1, wobei der Transport- und Verpackungsbehälter
insgesamt kastenförmigen Transport- und Verpackungsbehälter ausgebildet ist und von zwei Segmenten ausgebildet ist, die jeweils in der Art eines Schubfachs ausgebildet sind, oder
eine Mehrzahl von Segmenten umfasst, die jeweils in der Art eines Schubfachs ausgebildet sind, wobei jeweils ein Segment über einem anderen Segment angeordnet werden kann und diese ineinander geschoben werden können, um gemeinsam den Transport- und Verpackungsbehälter in einer gestapelten Anordnung von mehreren Segmenten auszubilden, aus der einzelne schubfachartige Segmente wieder abgezogen werden können

3. Transport- und Verpackungsbehälter nach Anspruch 1 oder 2, wobei in Bereichen von Seitenwänden der Segmente (320, 330), in denen Ränder oder Eckbereiche der Seitenwände im zusammengesetzten oder zusammengesteckten Zustand der Segmente einander gegenüberliegen oder in Anlage sind, diese Ränder oder Eckbereiche elastische Eigenschaften aufweisen, um die elastischen Dichtungsmittel (327, 328, 334, 335) auszubilden, wobei die elastischen Dichtungsmittel (327, 328, 334,335)
durch Wahl von Materialeigenschaften in den Bereichen von Seitenwänden der Segmente (320, 330), in denen die Ränder oder Eckbereiche der Seitenwände im zusammengesetzten oder zusammengesteckten Zustand der Segmente einander gegenüberliegen oder in Anlage sind, vorgegeben sind, oder
durch Anordnen von elastischen Dichtungslippen in den Bereichen von Seitenwänden der Segmente (320, 330) ausgebildet sind, in denen die Ränder oder Eckbereiche der Seitenwände im zusammengesetzten oder zusammengesteckten Zustand der Segmente einander gegenüberliegen oder in Anlage sind.

4. Transport- und Verpackungsbehälter nach Anspruch 3, wobei die elastischen Dichtungslippen
als separate Dichtungselemente in Nuten eingebracht sind, die in den Bereichen von Seitenwänden der Segmente (320, 330) angeordnet sind, in denen die Ränder oder Eckbereiche der Seitenwände im zusammengesetzten oder zusammengesteckten Zustand der Segmente einander gegenüberliegen oder in Anlage sind, oder
mittels eines 2K-Spritzgussverfahrens aus einem weicheren elastischen Kunststoff in den Bereichen von Seitenwänden der Segmente (320, 330) ausgebildet sind, in denen die Ränder oder Eckbereiche der Seitenwände im zusammengesetzten oder zusammengesteckten Zustand der Segmente einander gegenüberliegen oder in Anlage sind.

5. Transport- und Verpackungsbehälter nach einem der vorhergehenden Ansprüche, wobei die Segmente jeweils als ausziehbare Schubfächer ausgebildet sind, die jeweils drei Seitenwände (321, 322; 331, 332) und einen Boden (323; 333) aufweisen.

6. Transport- und Verpackungsbehälter nach einem der vorhergehenden Ansprüche, wobei am unteren Rand der Seitenwände des jeweiligen Segments zumindest eine weitere Ausnehmung (336) oder ein weiterer Vorsprung (328) ausgebildet ist, die oder der sich in Längsrichtung und parallel zu den an den Seitenwänden vorgesehenen und korrespondierend zueinander ausgebildeten Führungsschienen (327) und Führungsausnehmungen (335) erstreckt und als zusätzliches Dichtungsmittel wirkt, um ein Eindringen von Verunreinigungen in den Innenraum des Transport- und Verpackungsbehälters zu verhindern, wobei
die weitere Ausnehmung (336) oder der weiterer Vorsprung (338) mit einer elastischen Kunststoffauflage versehen sein, insbesondere ausgebildet in einer 2K-Spritzgusstechnik, sodass die weitere Ausnehmung (336) oder der weitere Vorsprung (328) gegen die jeweils zugeordnete Führungsschiene (327) oder Führungsausnehmung (335) elastisch verspannt ist, um den Innenraum des Transport- und Verpackungsbehälters gezielt auch in diesen Bereichen abzudichten.

7. Transport- und Verpackungsbehälter nach einem der vorhergehenden Ansprüche, wobei
die Segmente (320, 330) unmittelbar miteinander verrastbar sind, um den Transport- und Verpackungsbehälter auszubilden,
wobei an den Segmenten (320, 330) Verriegelungsmittel vorgesehen sind, um die Segmente im zusammengesetzten oder zusammengesteckten Zustand miteinander zu verriegeln.

8. Transport- und Verpackungsbehälter nach einem der vorhergehenden Ansprüche, wobei die Positionierungsmittel (324) unmittelbar an zumindest einem der Segmente ausgebildet sind.

9. Transport- und Verpackungsbehälter nach einem der Ansprüche 1 bis 7, wobei die Positionierungsmittel (25) von einer Mehrzahl von Aufnahmen (39) ausgebildet sind, die in einem Träger ausgebildet sind, der in den Transport- und Verpackungsbehälter (1) einsetzbar und aus diesem wieder herausnehmbar ist, wobei der Träger so ausgelegt ist, dass die Behälter in die zugeordneten Aufnahmen einführbar sind, wobei die Aufnahmen sich in Längsrichtung der Behälter erstrecken und ausgelegt sind, um Seitenwandabschnitte (4) der Behälter (2) zumindest abschnittsweise geklemmt zu halten, wobei die Aufnahmen von durchgehend ausgebildeten Seitenwänden ausgebildet sind und Öffnungsweiten der Aufnahmen (39) zwischen einer ersten Stellung, in welcher die Behälter in die Aufnahmen einführbar sind, und einer zweiten Stellung, in welcher die Behälter geklemmt sind, durch koordiniertes Verstellen der Seitenwände der Aufnahmen verstellbar sind.

10. Transport- und Verpackungsbehälter nach Anspruch 9, wobei der Träger (25) und/oder zumindest die Aufnahmen (39) des Trägers (25) und/oder zumindest ein Boden (320) des Transport- und Verpackungsbehälters (1) aus einem hochtemperaturfesten Kunststoff ausgebildet ist, der Temperaturen von bis zu 330°C oder von bis 350 °C resistiert.

11. Transport- und Verpackungsbehälter nach einem der Ansprüche 1 bis 9, wobei zumindest ein Boden (320) des Transport- und Verpackungsbehälters (1) aus einem Metall ausgebildet ist, wobei das Metall mit einem Basisbeschichtungsmaterial bestehend aus SiO₂, TiO₂, Al₂O₃ oder ZrO₂ beschichtet und anschließend getempert oder gebrannt ist.

12. Transport- und Verpackungsbehälter nach einem der vorhergehenden Ansprüche, wobei Seitenwände des Transport- und Verpackungsbehälters zumindest abschnittsweise mit Durchbrechungen (326; 329) versehen sind, die mittels einer aufgeklebten gasdurchlässigen Kunststofffolie verschlossen sind.

13. Verfahren zur thermischen Behandlung von Behältern (2) für Substanzen für medizinische, pharmazeutische oder kosmetische Anwendungen, unter Verwendung eines Transport- und Verpackungsbehälters (1) nach einem der vorhergehenden Ansprüche und/oder eines Trägers (25) mit Positionierungsmitteln nach einem der Ansprüche 8 oder 9, wobei der Boden (320) des Transport- und Verpackungsbehälters (1) mit einer Mehrzahl von darin aufgenommenen zylindrischen Behältern (2) oder der Träger (25) mit einer Mehrzahl von diesem gehaltenen zylindrischen Behältern (2) in einer Wärme-Prozessierungsstation mit Temperaturen von bis zu 330°C oder von bis zu 350°C prozessiert wird, wobei die zumindest zwei Segmente (320, 330) zusammengesetzt oder zusammengesteckt werden, um gemeinsam den Transport- und Verpackungsbehälter (1) auszubilden, wobei die elastischen Dichtungsmittel (327, 328, 334, 335, 130) den Innenraum des Transport- und Verpackungsbehälters (1) gegen die Umgebung abdichten.

14. Verfahren nach Anspruch 13, mit den weiteren Schritten:
Anordnen der Mehrzahl von Behältern (2) in dem Transport- und Verpackungsbehälter (1), um die Behälter durch die Positionierungsmittel (324; 25) im Innenraum des Transport- und Verpackungsbehälters (1) in einer regelmäßigen Anordnung so zu positionieren, dass eine Berührung von unmittelbar benachbarten Behältern (2) verhindert ist; und
Prozessierung der Mehrzahl von Behältern (2), während diese in dem Transport- und Verpackungsbehälter (1) oder in einem Segment (320, 330) davon angeordnet sind, in einer Wärme-Prozessierungsstation mit Temperaturen von bis zu 330°C oder von bis zu 350°C.

15. Verfahren nach Anspruch 13 oder 14, mit den weiteren Schritten:
Öffnen des Transport- und Verpackungsbehälters (1) durch Verschieben oder Freigeben von zumindest einem Segment der Mehrzahl von Segmenten (320, 330) des Transport- und Verpackungsbehälters (1), sodass der Innenraum des Transport- und Verpackungsbehälters (1) zur Entnahme des Trägers (25) mit der Mehrzahl der von diesem gehaltenen Behälter (2) zugänglich ist;
Herausnehmen des Trägers (25) mit der Mehrzahl der von diesem gehaltenen Behälter (2);
Prozessierung der Mehrzahl von Behältern (2), während diese von dem Träger (25) gehalten sind, in der Wärme-Prozessierungsstation mit Temperaturen von bis zu 330°C oder von bis zu 350°C;
Anordnen des Trägers (25) mit der Mehrzahl der von diesem gehaltenen Behälter (2) in oder auf zumindest einem der Segmente; und
Zusammensetzen oder Zusammenstecken der Segmente (320, 330), sodass diese gemeinsam den Transport- und Verpackungsbehälter (1) ausbilden und die elastischen Dichtungsmittel (327, 328, 334, 335, 130) den Innenraum des Transport- und Verpackungsbehälters (1) gegen die Umgebung abdichten.

16. Verfahren nach einem der Ansprüche 13 bis 15, wobei die Prozessierung der Mehrzahl von Behältern (2) in einem Heißofen oder Heißtunnel mit Temperaturen von bis zu 330°C oder von bis zu 350°C so erfolgt,
dass Endotoxine und/oder die Lyse von Bakterien und/oder Cytokine induzierende Substanzen (CIS) in den Behältern (2) thermisch entfernt werden, oder
dass Silikonschichten auf Innenoberflächen der Behälter (2) eingebrannt werden.

## Claims

1. A transport and packaging container for accommodating a plurality of cylindrical containers (2) for substances for medical, pharmaceutical or cosmetic applications, comprising at least two segments (320, 330) of which each can be handled separately and which can be assembled or stuck together to jointly form the transport and packaging container (1), wherein
a first segment (330; 320) of the at least two segments has a bottom (333; 323) for supporting the plurality of containers (2), and
positioning devices (324; 25) are provided for positioning the plurality of containers (2) in the interior of the transport and packaging container (1) in a regular arrangement in such a manner that a collision of the directly adjacent containers (2) is prevented, wherein
resilient sealing means (327, 328, 334, 335) are provided at the segments (320, 330) so that the segments (320, 330) can be assembled or stuck together to the transport and packaging container repeatedly and so that the interior of the transport and packaging container (1) is sealed against the environment,
**characterized in that** guide rails (327) and guide recesses (335) are provided at the segments, which are formed corresponding to each other and which guide the segments for assembling the transport and packaging container (1), wherein
the guide rails (327) or guide recesses (335) are surrounded by sealing lips that prevent contaminants from entering the interior of the transport and packaging container, or
the guide rails (327) or guide recesses (335) are provided with a resilient plastic plating, which is particularly formed by means of a two-component injection molding technology, so that the resilient plastic plating is pressed resiliently against opposite side walls of the guide rails or guide recesses of the other segment in order to seal the interior of the transport and packaging container specifically also in these areas.

2. The transport and packaging container according to claim 1, wherein the transport and packaging container
overall is formed as a box-shaped transport and packaging container and is formed by two segments each of which is formed as a drawer, or
comprises a plurality of segments respectively formed in the manner of a drawer,
wherein respectively one segment can be disposed above another segment and these can be inserted into each other for jointly forming the transport and packaging container in a stacked arrangement consisting of a plurality of segments, from which individual drawer-like segments can be removed again.

3. The transport and packaging container according to claim 1 or 2, wherein in regions of side walls of the segments (320, 330), where edges or corner regions of the side walls are opposite to each other or in contact with each other, when the segments are assembled or stuck together to form the transport and packaging container, these edges or corner regions have resilient characteristics to form the resilient sealing means (327, 328, 334, 335, wherein the resilient sealing means (327, 328, 334, 335)
are provided by the choice of material properties in the regions of side walls of said segments (320, 330), where edges or comer regions of the side walls are opposite to each other or in contact with each other, when the segments are assembled or stuck together, or
are formed by disposing resilient sealing lips in the regions of side walls of the segments (320, 330), where edges or corner regions of the side walls are opposite to each other or in contact with each other, when the segments are assembled or stuck together.

4. The transport and packaging container according to claim 3, wherein the resilient seal lips
are inserted as separate sealing members into grooves provided in the regions of side walls of the segments (320, 330), where the edges or corner regions of the side walls are opposite to each other or in contact with each other, when the segments are assembled or stuck together, or
are formed by means of a two-component-injection-molding process of a softer resilient plastic in the regions of side walls of the segments (320, 330), where the edges or corner regions of the side walls are opposite to each other or in contact with each other, when the segments are assembled or stuck together.

5. The transport and packaging container according to any of the preceding claims, wherein the segments are each formed as drawers that can be pulled out, each having three side walls (321; 322; 331, 332) and a bottom (333; 323).

6. The transport and packaging container according to any of the preceding claims, wherein at least one additional recess (336) or at least one additional protrusion (328) is formed along a bottom edge of the side walls of the respective segment, which extends in the longitudinal direction and in parallel to the guide rails (327) and guide recesses (335) that are provided at the side walls and that are formed corresponding to each other and act as additional sealing members for preventing contaminants from entering the interior of the transport and packaging container, wherein
the additional recess (336) or additional protrusion (328) is provided with a resilient plastic plating, which is particularly formed by means of a two-component injection molding technology, so that the additional recess (336) or the additional protrusion (328) is pressed resiliently against the respectively associated guide rail (327) or guide recess (335) in order to seal the interior of the transport and packaging container specifically also in these areas.

7. The transport and packaging container according to any of the preceding claims, wherein
the segments (320, 330) can be locked directly to one another to form the transport and packaging container,
wherein locking means are provided on the segments (320, 330) to lock the segments, when the segments are assembled or stuck together.

8. The transport and packaging container according to any of the preceding claims, wherein the positioning devices (324) are formed directly on at least one of the segments.

9. The transport and packaging container according to any of claims 1 to 7, wherein the positioning devices (25) are formed by a plurality of receptacles (39), which are formed in a carrier that can be inserted into and removed out of the transport and packaging container (1), said carrier being configured such that the containers can be inserted into the associated receptacles, wherein the receptacles extend in the longitudinal direction of the containers and are configured to support sidewall portions (4) of the containers (2) at least partially clamped, wherein the receptacles are formed by continuous side walls and wherein the opening widths of the receptacles (39) can be adjusted by coordinated adjustment of the side walls between a first position, in which the containers can be inserted into the receptacles, and a second position, in which the containers are clamped.

10. The transport and packaging container according to claim 9, wherein the carrier (25) and/or at least the receptacles (39) of the carrier (25) and/or at least a bottom (320) of the transport and packaging container (1) is formed of a high-temperature-resistant plastic material, which is resistant to high temperatures of up to 330 °C or up to 350 °C.

11. The transport and packaging container according to any of claims 1 to 9, wherein at least a bottom (320) of the transport and packaging container (1) is formed of a metal, wherein the metal is coated by a basic coating material consisting of SiO₂, TiO₂, Al₂O₃ or ZrO₂ and then tempered or burned.

12. The transport and packaging container according to any of the preceding claims, wherein side walls of the transport and packaging container are provided at least partially with openings (326; 329), which are closed by bonding a gas-permeable plastic foil.

13. A process for the thermal treatment of containers (2) for substances for medical, pharmaceutical or cosmetic applications, using a transport and packaging container (1) according to any of the preceding claims and/or a carrier (25) comprising positioning devices according to any of claims 8 or 9, wherein the bottom (320) of the transport and packaging container (1) together with a plurality of cylindrical containers (2) accommodated therein or the carrier (25) together with a plurality of cylindrical containers (2) supported by it are processed in a thermal processing station at temperatures of up to 330 °C or up to 350 °C, wherein the at least two segments (320, 330) are assembled or stuck together to jointly form the transport and packaging container (1), wherein the resilient sealing means (327, 328, 334, 335, 130) seal the interior of the transport and packaging container (1) against the environment.

14. The process according to claim 13, further comprising:
disposing the plurality of containers (2) in the transport and packaging container (1) for positioning the containers by means of the positioning devices (324; 25) in the interior of the transport and packaging container (1) in a regular array so that collisions of directly adjacent containers (2) are prevented; and
processing the plurality of containers (2) while they are arranged in the transport and packaging container (1) or in a segment (320, 330) thereof in a thermal processing station at temperatures of up to 330 °C or up to 350 °C.

15. The process according to claim 13 or 14, further comprising:
opening the transport and packaging container (1) by displacing or releasing at least one segment of said plurality of segments (320, 330) of the transport and packaging container (1) so that the interior of the transport and packaging container (1) is accessible for the removal of the carrier (25) together with the plurality of containers (2) supported by it;
removing the carrier (25) together with the plurality of containers (2) supported by it;
processing of the plurality of containers (2) while they are supported by the carrier (25) in the thermal processing station at temperatures of up to 330 °C or up to 350 °C;
placing the carrier (25) together with the plurality of containers (2) supported by it in or on at least one of the segments; and
assembling of or sticking together the segments (320, 330), so that they together form the transport and packaging container (1) and so that the resilient sealing means (327, 328, 334, 335, 130) seal the interior of the transport and packaging container (1) against the environment.

16. The process according to any of claims 13 to 15, wherein the processing of the plurality of containers (2) is performed in a hot oven or hot tunnel at temperatures of up to 330 °C or up to 350 °C,
so that endotoxins and/or the lysis of bacteria and/or cytokine inducing substances (CIS) are removed thermally in the containers (2), or
so that silicone layers are burned-in on inner surfaces of the containers (2).

## Revendications

1. Récipient d'emballage et de transport destiné à recevoir une pluralité de récipients cylindriques (2) pour des substances pour des applications médicales, pharmaceutiques ou cosmétiques, comprenant au moins deux segments (320, 330) pouvant chacun être séparément manipulés et pouvant être assemblés ou emboîtés en même temps pour former conjointement le récipient d'emballage et de transport (1), dans lequel,
un premier segment (330; 320) parmi le ou les segments dispose d'un fond (333, 323) pour supporter la pluralité de récipients (2), et
des dispositifs de positionnement (324; 25) prévus pour disposer la pluralité de récipients (2) à l'intérieur du récipient d'emballage et de transport (1) suivant une disposition régulière de telle manière à éviter toute collision entre deux récipients directement adjacents, dans lequel
des moyens d'étanchéité élastiques (327, 328, 334, 335) sont prévus au niveau des segments (320, 330) de sorte que les segments (320, 330) peuvent être assemblés ou emboîtés ensemble répétitivement à l'intérieur du récipient d'emballage et de transport et de telle manière que l'intérieur du récipient d'emballage et de transport (1) est rendue étanche par rapport à l'environnement,
**caractérisé en ce que** des rails de guidage (327) et les évidements de guidage (335) sont prévus pour les segments, et sont formés en correspondance les uns par rapport aux autre et permettent le guidage des segments pour l'assemblage du récipient d'emballage et de transport (1), dans lequel
les rails de guidage (327) ou les évidements de guidage (335) sont entourés de lèvres d'étanchéité empêchant la pénétration d'agent contaminant à l'intérieur du récipient d'emballage et de transport, ou
les rails de guidage (327) ou les évidements de guidage (335) sont prévus avec un recouvrement plastique élastique, qui est formé en particulier au moyen d'une technique de moulage par injection à deux composants, de manière à ce que le revêtement plastique élastique est pressé de manière élastique contre les parois latérales opposées des rails de guidage ou des évidements de guidage de l'autre segment afin d'étanchéité également particulièrement l'intérieur des récipients d'emballage et de transport dans ces zones.

2. Le récipient d'emballage et de transport selon la revendication 1, dans lequel le récipient d'emballage et de transport
se présente partout sous la forme d'une boîte et est formé par deux segments prenant chacun la forme d'un tiroir, ou
comporte une pluralité de segments respectivement de forme d'un tiroir, dans lequel un segment, respectivement peut être disposé au-dessus d'un autre segment qui peuvent être insérés l'un dans l'autre pour former conjointement le récipient d'emballage et de transport dans une disposition en pile consistant en une pluralité de segments, à partir desquels des segments en forme de tiroirs peuvent être extraits à nouveau.

3. Le récipient d'emballage et de transport selon la revendication 1 ou 2, dans lesquels dans les zones des parois latérales des segments (320, 330), ou les bords ou les régions d'angles des parois latérales sont opposées l'une à l'autre ou sont en contact l'une avec l'autre, lorsque les segments sont assemblés ou emboîtés ensemble pour former le récipient d'emballage et de transport, ces bords ou zones d'angles présentent des caractéristiques élastiques pour former les moyens d'étanchéités élastiques (327, 328, 334, 335) dans lequel les moyens d'étanchéité élastiques (327, 328, 334, 335)
sont prévus par un choix des propriétés de matériau dans les régions des parois latérales desdits segments (320, 330), ou les bords ou régions d'angles des parois latérales sont opposées l'une à l'autre ou en contact l'une avec l'autre, lorsque les segments sont assemblés ou emboîtés ensemble, ou
sont formés en disposant des lèvres d'étanchéité élastiques dans les régions des parois latérales des segments (320, 330), dans lesquels les bords ou régions d'angles des parois latérales sont opposées l'une à l'autre ou en contact l'une avec l'autre, lorsque les segments sont assemblés ou emboîtés ensemble.

4. Le récipient d'emballage et de transport selon la revendication 3, dans lequel les lèvres d'étanchéité élastiques
sont disposées sous forme d'éléments d'étanchéité séparés dans des rainures disposées dans les régions des parois latérales des segments (320, 330), ou les bords ou régions d'angle des parois latérales sont opposées l'une à l'autre ou en contact l'une avec l'autre, lorsque les segments sont assemblés ou emboîtés ensemble, ou
sont formées au moyen d'un procédé de moulage 2K-injection à partir d'une matière plastique élastique plus molle dans les zones de parois latérales des segments (320, 330), dans lequel les bords ou les régions de coin des parois latérales dans l'état assemblé ou assemblé des segments sont opposées l'une à l'autre ou en butée.

5. Le récipient d'emballage et de transport selon l'une des revendications précédentes, dans lequel les segments sont formés chacun en tant que tiroirs coulissants, chacun ayant trois parois latérales (321, 322 ; 331, 332) et une base (333, 323).

6. Le récipient d'emballage et de transport selon l'une des revendications précédentes, dans lequel au moins un évidemment additionnel (326) ou au moins une protubérance supplémentaire (328) est formées le long d'un bord de fond des parois latérales du segment respectif, qui s'étendent dans la direction longitudinale et parallèle aux rails de guidage (327) et aux évidements de guidage (335) qui sont prévus sur les parois latérales et qui sont formées en correspondance les uns aux autres et opèrent comme des éléments d'étanchéité additionnels pour empêcher l'intrusion des contaminateurs à l'intérieur du récipient d'emballage et de transport, dans lequel
l'évidemment additionnel (336) ou la protubérance supplémentaire (328) sont prévues avec un recouvrement plastique élastique, qui est formé en particulier au moyen d'un procédé de moulage, de telle manière que l'évidemment additionnel (336) ou la protubérance supplémentaire (328) sont pressées de manière élastique contre le raid de guidage respectif associé (327) ou l'évidemment de guidage (335) afin de rendre également spécifiquement étanche l'intérieur du récipient d'emballage ou de transport dans ces zones.

7. Le récipient d'emballage et de transport selon l'une des revendications précédentes, dans lequel
les segments (320, 330) peuvent être verrouillés directement les uns aux autres pour former le conteneur de transport et d'emballage,
dans lequel des moyens de verrouillage sont prévus sur les segments (320, 330) pour verrouiller les segments lorsque les segments sont assemblés ou emboîtés.

8. Le récipient d'emballage et de transport selon l'une des revendications précédentes, dans lequel les moyens de positionnement (324) sont formées directement sur au moins l'un des segments.

9. Le récipient d'emballage et de transport selon l'une des revendications 1 à 7, dans lequel les moyens de positionnement (25) sont formés d'une pluralité de réceptacles (39), formés dans un support qui peut être inséré ou enlevé du récipient d'emballage et de transport (1), ledit support étant configuré de telle manière que les récipients peuvent être insérés dans leurs réceptacles associés, dans lequel les réceptacles se prolongent suivant une direction longitudinale des récipients et sont configurés pour soutenir des parties latérales (4) des récipients (2), au moins partiellement serrés, dans lequel les réceptacles sont formés par des parois latérales continues et dans lesquels les largeurs d'ouverture des réceptacles (39) peuvent être ajustées par un réglage coordonné des parois latérales entre une première position, dans laquelle les récipients peuvent être insérés dans les réceptacles, et une seconde position, dans laquelle les récipient sont serrés.

10. Le récipient d'emballage et de transport selon la revendication 9, dans lequel le support (25) et / ou au moins les réceptacles (39) du support (25) et / ou au moins un fond (320) du récipient d'emballage et de transport (1) est formé d'un matériau plastique résistant à haute température, résistant à des températures allant jusqu'à 330 ° C ou à 350 ° C .

11. Le récipient d'emballage et de transport selon l'une quelconque des revendications 1 à 9, dans lequel au moins un fond (320) du récipient d'emballage et de transport (1) est formé de métal, dans lequel le métal est recouvert d'un matériau de revêtement de base constitué de SiO₂, TiO₂, Al₂O₃ ou ZrO₂ et ensuite trempé ou brûlé.

12. Le récipient d'emballage et de transport selon l'une des revendications précédentes, dans lequel les parois latérales du récipient d'emballage et de transport sont pourvus au moins partiellement avec des ouvertures (326; 329), qui sont fermées par collage d'un film plastique perméable au gaz.

13. Un procédé pour le traitement thermique de récipients (2) pour des substances destinées à des applications cosmétiques, pharmaceutiques ou médicales, en utilisant un récipient d'emballage et de transport (1) selon l'une quelconque des revendications précédentes et/ou d'un support (25) comprenant des moyens de positionnement selon l'une quelconque des revendications 8 à 9, dans lequel le fond (320) du récipient d'emballage et de transport (1) et une pluralité de récipients cylindriques (2) y logés, ou le support (25) avec une pluralité de récipients cylindriques (2) supportés par lui, sont traités au sein d'une station de traitement thermique à des températures allant jusqu'à 330 ° C ou jusqu'à 350 ° C, dans lequel les deux segments au moins (320, 330) sont assemblés ou emboîtés pour former ensemble le récipient d'emballage et de transport (1), dans lequel les moyens d'étanchéité élastiques (327 , 328, 334, 335, 130) permettent l'étanchéité de l'intérieur du récipient d'emballage et de transport (1) vis à vis de l'environnement.

14. Le procédé selon la revendication 13, comprenant en outre :
disposer la pluralité de récipients (2) dans le récipient d'emballage et de transport (1) pour le positionnement suivant une disposition régulière des récipients au moyen des moyens de positionnement (324; 25) au sein du récipient d'emballage et de transport (1) afin d'éviter la collision de récipients directement adjacents (2) ; et
permettre le traitement de la pluralité des récipients (2), tandis qu'ils sont disposés dans le récipient d'emballage et de transport (1) ou dans un segment (320, 330) de celui-ci au sein d'une station de traitement thermique à des températures allant jusqu'à 330 ° C ou jusqu'à 350 ° G

15. Le procédé selon la revendication 13 ou 14, comprenant en outre :
l'ouverture du récipient d'emballage et de transport (1) par un déplacement ou une libération d'au moins un segment de la pluralité de segments (320, 330) du récipient d'emballage et de transport (1) en sorte que l'intérieur du récipient d'emballage et de transport (1) soit accessible pour le retrait du support (25) ensemble avec la pluralité de récipients (2) supportés par celui-ci ;
le retrait du support (25) avec la pluralité de récipients (2);
le traitement de la pluralité de récipients (2), tandis qu'ils sont soutenus par le support (25) dans la station de traitement thermique à des températures allant jusqu'à 330 ° C ou jusqu'à 350 ° C;
positionner le support (25) avec la pluralité de récipients (2) supportés par lui au sein d'un ou sur un segment au moins ; et
assembler ou accoupler ensemble les segments (320, 330), de telle manière à former ensemble le récipient d'emballage et de transport (1) et de telle manière que les moyens d'étanchéité élastiques (327, 328, 334, 335, 130) assurent l'étanchéité du récipient d'emballage et de transport (1) relativement à l'environnement.

16. Le procédé selon l'une quelconque des revendications 13 à 15, dans lequel le traitement de la pluralité de récipients (2) est effectué au sein d'un four chaud ou au sein d'un tunnel chaud à des températures allant jusqu'à 330 ° C ou jusqu'à 350 ° C, de telle manière que les endotoxines et / ou la lyse des bactéries et / ou des cytokines induisant des substances (CIS) peuvent être enlevés thermiquement dans les récipients (2), ou
que les couches de silicone sont cuits sur des surfaces intérieures du récipient (2).
